# EUROPEAN PATENT SPECIFICATION

(11) **EP 4 413 158 B1**
(45) Date of publication and mention of the grant of the patent: **30.09.2026**
(21) Application number: 22879350.1
(22) Date of filing: 07.10.2022
(51) Int. Cl.: C12Q 1/6834, C12N 15/10, C12N 15/90, C12Q 1/6837, C12Q 1/6841, C12Q 1/6874, C12Q 1/6876

(54) **B(EAD-BASED) A(TACSEQ) P(ROCESSING)**
B(EAD-BASIERTE) A(TACSEQ) P(ROZESSING)
TRAITEMENT ATACSEQ À BASE DE BILLES

(30) Priority: 08.10.2021 US 202163253977 P
(43) Date of publication of application: 14.08.2024
(73) Proprietor: Bio-Rad Laboratories, Inc., Hercules, CA 94547 (US); President and Fellows of Harvard College, Cambridge, MA 02138 (US)
(72) Inventor: BUENROSTRO, Jason, Cambridge, Massachusetts 02138 (US); LEBOFSKY, Ronald, Hercules, California 94547 (US)
(74) Representative: dompatent
(86) International application number: PCT/US2022/046116
(87) International publication number: WO 2023/059917

(56) References cited:
- WO-A1-2012/061832
- WO-A1-2020/041293
- WO-A1-2021/061841
- WO-A2-2020/123301
- US-A1- 2020 056 231
- CHEN HE ET AL: "Tagmentation on Microbeads: Restore Long-Range DNA Sequence Information Using Next Generation Sequencing with Library Prepared by Surface-Immobilized Transposomes", vol. 10, no. 14, 15 March 2018 (2018-03-15), United States, pages 11539 - 11545, XP055915782, ISSN: 1944-8244, Retrieved from the Internet <URL:https://pubs.acs.org/doi/pdf/10.1021/acsami.8b01560> DOI: 10.1021/acsami.8b01560

## Description

### BACKGROUND OF THE INVENTION

Tagging biological substrates with molecular barcodes in partitions can provide novel biological insight of the substrates that co-localize to discrete partitions, through the sequencing of the molecular barcodes and analysis, thereof. Increasing the number of barcoding competent partitions, such as droplets, increases the number of sequencing based data points and converts a greater fraction of input substrates into data. Barcodes can be delivered to partitions, such as droplets, using beads as the delivery vehicle. Thus, barcode bead overloading in partitions, which results in partitions with more than one bead and increases the percentage of barcoding competent partitions, provides higher substrate to sequencing data conversion rates. However, when two or more barcodes occur in discrete partitions, the substrates and data are split between the two barcodes, creating fractionated data points.

In A(ssay for) T(ransposase) A(ccessisble) C(hromatin) (by) Seq(uencing) and related applications that use transposases to add adapter oligonucleotides, immobilized clonal barcode oligonucleotides on solid supports are used to tag transposed double-stranded DNA fragments, DNA and/or ds-cDNA. The target unit may include a single cell and/ or a group of cells. It may also include a spatially defined cell on a 2D planar substrate and / or it may include a spatially defined group of cells on a 2D planar substrate. Although PCR can be used to tag substrates with clonal barcodes, one-step tagging biochemistries are preferred and / or are only feasible in some embodiments where thermal cycling is not possible. One-step tagging biochemistries may include hybridization, hybridization plus ligation, and/or hybridization plus primer templated nucleic acid synthesis. One application where thermal cycling is not desired is in single cell analysis where barcoding is carried out through hybridization only to minimize enzyme costs in massively parallel partitions that represent significant volume when taken together. Another application where thermal cycling is difficult is in spatial ATAC-Seq analysis as 2D arrays are not easily amenable to efficient thermal cycling without drying the reaction components.

### BRIEF SUMMARY OF THE INVENTION

Where >2 clonal barcodes tag a target unit using a one-step biochemistry, whether that is a cell and/or a group of cells and/or a spatially defined cell and/or a spatially defined group of cells, it is currently unknown how to use sequencing data, without *a priori* knowledge of the clonal barcodes contributing to the tag event, to annotate co-barcoding multiple clonal barcodes that tag the same target unit. Knowledge of the multiple barcodes that tag the same target unit is desirable to unify single cell data that would otherwise be fractionated amongst unannotated clonal barcodes and/or to create a spatial map of clonal barcodes without a priori knowledge of their spatial 2D positions. Although *a priori* knowledge of spatial 2D positions is feasible, it requires significant and costly pre-processing by either directed positioning of the clonal barcodes and / or pre-sequencing of the clonal barcodes prior to barcoding the target units. Although tertiary substrates that are neither the clonal barcode or the target unit can be used to annotate co-barcoding clonal barcodes, this requires additional reagents, processing and sequencing that add to workflow time and costs. On the other hand, methods for deconvoluting sequencing reads from partitions that comprise performing tagmentation of nucleic acids in permeabilized cells, thereby forming at least one cleavage site in a target nucleic acid from one of the cells, are known from WO 2012/061832, US 2020/00562311 A1 and WO 2020/123301.

Here, we describe a substrate composition and method to use unique transposase cut signatures on different sequenced fragments to annotate clonal barcodes that co-barcode the same target unit. This does not require the use of thermal cycling and is compatible with hybridization-only biochemistries that optionally do not use enzymes. Also, this does not require *a priori* annotation of the clonal barcodes that co-barcode the target units. Although the methods described herein can be used for single cell and / or spatial ATACseq applications, it can also be used for any single cell and / or spatial analyses where a transposase is used to process the substrate upstream of clonal barcoding, such as RNAseq, TotalRNAseq, MethylSeq, DNAseq, HiCSeq, proteinSeq, and combinations thereof. Nuclei, as well as cells can constitute the target units.

As a first aspect, the present invention provides methods of deconvoluting sequencing reads from partitions as set forth in claims 1 and 6. In particular, provided is a method comprising,
performing tagmentation of nucleic acids in permeabilized cells in a mixture, thereby forming at least one cleavage site in a target nucleic acid from one of the cells to form a first nucleic acid fragment and a second nucleic acid fragment, wherein the first and second nucleic acid fragments have at the cleavage site a single-stranded 9 nucleotide sequence, which are complementary to each other, linked to a transposase oligonucleotide delivered by a tagmentation transposase, wherein the transposase oligonucleotide has a double-stranded portion, and a single-stranded 5' portion comprising a universal sequence;
forming a plurality of partitions from the mixture and a plurality of beads and the permeabilized cells, wherein one of the partitions comprise the first nucleic acid fragment and second nucleic acid fragment and at least two beads, wherein the beads are linked to 5' ends of a plurality of clonal barcoding oligonucleotides, the barcoding oligonucleotides comprising a 5' PCR handle sequence, a 3' capture sequence and a barcode sequence unique to the bead to which the barcode oligonucleotide is linked, wherein the 3' capture sequence comprises a copy of said universal sequence (optionally instead the clonal barcoding oligonucleotides can be delivered to the partitions in droplets rather than attached to beads);
gap-filling the single-stranded 5' portion of the transposase oligonucleotide to form a reverse complement of the 5' portion and gap-filling the 9 nucleotide sequences, wherein the gap filling comprises using a polymerase to insert nucleotides using the single stranded sequences as a template;
hybridizing the 3' capture sequence of different barcoding oligonucleotides from different beads to the reverse complement of the 5' portion on the first and second nucleic acid fragments and extending the 3' capture sequence of the different barcoding oligonucleotides in a template-dependent manner with a polymerase to form barcoded first and second nucleic acid fragments; optionally combining the partitions into a bulk solution;
amplifying the barcoded first and second nucleic acid fragments with primers that hybridize to the PCR handle sequences;
generating sequencing reads from the amplified barcoded first and second nucleic acid fragments, wherein the sequencing reads include the barcode sequence, the 9 nucleotide sequence and at least a portion of the nucleic acid fragment from the cell;
identifying in the sequence reads the genomic location relative to the nucleic acid fragment and sequence identity of the 9 nucleotide sequence; and
determining sequencing reads having barcodes from the amplified barcoded first and second barcoding oligonucleotides were from the same partition if the 9 nucleotide sequences in the sequencing reads are reverse complementary sequences and the 9 nucleotide sequences in the sequencing reads are 5' to adjacent genomic positions.

In some embodiments, the nucleic acids in the permeabilized cells are chromosomal DNA and different chromosomal sequences differ in how accessible the different chromosomal sequences are to the transposase.

In some embodiments, the nucleic acids in the permeabilized cells have been stripped of histones.

In some embodiments, the single-stranded 5' portion of the transposase oligonucleotide comprises (ii) a unique molecular identifier barcode sequence. In some embodiments, the unique molecular barcode sequence is 4-10 bp long.

In some embodiments, the single-stranded 5' portion of the transposase oligonucleotide comprises a multiplexing identifier sequence that distinguishes different samples. In some embodiments, the multiplexing identifier sequence is 4-10 bp long.

In some embodiments, the nucleic acids in permeabilized cells are DNA. In some embodiments, the method comprises forming first strand cDNAs or double-stranded cDNAs in the permeabilized cells and the nucleic acids comprise cDNA. In some embodiments, the DNA is cellular genomic DNA.

In some embodiments, the partitions are droplets in an water-in-oil emulsion. In some embodiments, the partitions are microwells.

In some embodiments, the tagging further comprises tagging nucleic acids in the cells such that two or more types of nucleic acids are tagged and subsequently sequenced. In some embodiments, the two types of nucleic acids are selected from the group consisting of genomic DNA or cDNA.

Also provided is a method comprising,
performing tagmentation of nucleic acids in permeabilized cells in a mixture, thereby forming at least one cleavage site in a target nucleic acid from one of the cells to form a first nucleic acid fragment and a second nucleic acid fragment, wherein the first and second nucleic acid fragments have at the cleavage site a single-stranded 9 nucleotide sequence, which are complementary to each other, linked to a transposase oligonucleotide delivered by a tagmentation transposase, wherein the transposase oligonucleotide has a double-stranded portion, and a single-stranded 5' portion having a 5' phosphorylated end;
forming a plurality of partitions from the mixture, bridging oligonucleotides, and a plurality of beads and the permeabilized cells, wherein one of the partitions comprise the first nucleic acid fragment and second nucleic acid fragment and at least two beads, wherein the beads are linked to 5' ends of a plurality of clonal barcoding oligonucleotides, the barcoding oligonucleotides comprising a 5' PCR handle sequence, a 3' capture sequence and a barcode sequence unique to the 3' to which the barcode oligonucleotide is linked, and wherein the bridging oligonucleotides comprise (i) a 3' end sequence complementary to the 3' capture sequence of the clonal barcoding oligonucleotides and (ii) a 5' end sequence complementary to the universal sequence of the single-stranded 5' portion of the transposase oligonucleotide(optionally instead the clonal barcoding oligonucleotides can be delivered to the partitions in droplets rather than attached to beads);
in the partitions, tagging the first nucleic acid fragment with a barcoding oligonucleotide from a first bead and tagging the second nucleic acid fragment with a barcoding oligonucleotide from a second bead, wherein the tagging comprises hybridizing the 3' capture sequences of the clonal barcoding oligonucleotides to the 3' end sequences of the bridging oligonucleotides and hybridizing the 5' end sequences of the bridging oligonucleotides to the single-stranded 5' portion delivered by the transposase to the first nucleic acid fragment and the second nucleic acid fragment, thereby forming barcoded first and second nucleic acids;
optionally combining the partitions into a bulk solution;
ligating the 3' capture sequences of the clonal barcoding oligonucleotides to the single-stranded 5' portion delivered by the transposase to the first nucleic acid fragment and the second nucleic acid fragment;
gap-filling the 9 nucleotide sequence and the single stranded portion of the bead oligo ligated to tranposase adapter, wherein the gap filling comprises using a polymerase to insert nucleotides using the single stranded sequences as a template;
amplifying the barcoded first and second nucleic acid fragments with primers that hybridize to the PCR handle sequences;
generating sequencing reads from the amplified barcoded first and second nucleic acid fragments, wherein the sequencing reads include the barcode sequence, the 9 nucleotide duplication sequence and at least a portion of the nucleic acid fragment from the cell;
identifying in the sequence reads the genomic location relative to the nucleic acid fragment and sequence identity of the 9 nucleotide sequence; and
determining sequencing reads having barcodes from the amplified barcoded first and second barcoding oligonucleotides were from the same partition if the 9 nucleotide sequences in the sequencing reads are reverse complementary sequences and the 9 nucleotide sequences in the sequencing reads are 5' to adjacent genomic positions.

In some embodiments, the nucleic acids in the permeabilized cells are chromosomal DNA and different chromosomal sequences differ in how accessible the different chromosomal sequences are to the transposase.

In some embodiments, the nucleic acids in the permeabilized cells have been stripped of histones.

In some embodiments, the partitions further contain a proteinase, surfactant or chaotropic agent.

In some embodiments, the ligating occurs in the partitions. In some embodiments, the partitions are combined after the ligating
In some embodiments, the method comprises combining the partitions into a bulk solution. In some embodiments, the ligating occurs in the bulk solution.

In some embodiments, the single-stranded 5' portion of the transposase oligonucleotide comprises (i) a sequence complementary to the 5' end sequences of the bridging oligonucleotides and (ii) a unique molecular identifier barcode sequence. In some embodiments, the unique molecular barcode sequences is 4-10 bp long.

In some embodiments, the nucleic acids in permeabilized cells are DNA. In some embodiments, the method comprises forming first strand cDNAs or double-stranded cDNAs in the permeabilized cells and the nucleic acids comprise cDNA. In some embodiments, the DNA is cellular genomic DNA.

In some embodiments, the partitions are droplets in an water-in-oil emulsion In some embodiments, the partitions are microwells.

In some embodiments, the tagging further comprises tagging nucleic acids in the cells such that two or more types of nucleic acids are tagged and subsequently sequenced. In some embodiments, the two types of nucleic acids are selected from the group consisting of genomic DNA orcDNA.

As a second aspect of the invention, provided are methods of determining relative position of beads on a solid support as set forth in claim 12. In particular, provided is a method comprising
providing a tissue section fixed to a solid support;
performing tagmentation of nucleic acids in the tissue section, thereby forming at least one cleavage site in a target nucleic acid within the tissue section to form a first nucleic acid fragment and a second nucleic acid fragment, wherein the first and second nucleic acid fragments receive at the cleavage site a single-stranded 9 nucleotide duplication sequence linked to a transposase oligonucleotide with a double-stranded portion and a single-stranded 5' portion delivered by the transposase;
contacting to the tagmented nucleic acid in the tissue section bridging oligonucleotides and oligonucleotides from a plurality of beads, wherein the beads are linked to 5' ends of a plurality of clonal barcoding oligonucleotides, the barcoding oligonucleotides comprising a 5' PCR handle sequence, a 3' capture sequence and a barcode sequence unique to the bead to which the barcode oligonucleotide is linked, wherein the oligonucleotides are released from the beads and wherein the bridging oligonucleotides comprise (i) a 3' end sequence complementary to the 3' capture sequence of the clonal barcoding oligonucleotides and (ii) a 5' end sequence complementary to the single-stranded 5' portion of the transposase oligonucleotide (optionally instead the clonal barcoding oligonucleotides can be delivered to the tissue in droplets rather than attached to beads);
tagging the first nucleic acid fragment with a barcoding oligonucleotide from a first bead and tagging the second nucleic acid fragment with a barcoding oligonucleotide from a second bead, wherein the tagging comprises hybridizing the 3' capture sequences of the clonal barcoding oligonucleotides to the 3' end sequences of the bridging oligonucleotides and hybridizing the 5' end sequences of the bridging oligonucleotides to the single-stranded 5' portion delivered by the transposase to the first nucleic acid fragment and the second nucleic acid fragment, thereby forming barcoded first and second nucleic acids;
optionally washing the barcoded first and second nucleic acids from the planar solid support; ligating the 3' capture sequences of the clonal barcoding oligonucleotides to the single-stranded 5' portion delivered by the transposase to the first nucleic acid fragment and the second nucleic acid fragment;
optionally washing the barcoded first and second nucleic acids from the planar solid support if the barcoded first and second nucleic acids have not been previously washed from the planar support;
gap-filling the 9 nucleotide sequence, wherein the gap filling comprises using a polymerase to insert nucleotides using the single stranded sequences as a template ;
amplifying the barcoded first and second nucleic acid fragments with primers that hybridize to the PCR handle sequences;
generating sequencing reads from the amplified barcoded first and second nucleic acid fragments, wherein the sequencing reads include the barcode sequence, the 9 nucleotide sequence and at least a portion of the nucleic acid fragment from the cell;
identifying in the sequence reads the genomic location relative to the nucleic acid fragment and sequence identity of the 9 nucleotide duplication sequence; and
determining sequencing reads having barcodes from the amplified barcoded first and second barcoding oligonucleotides were from adjacent beads on the tissue section if the 9 nucleotide sequences in the sequencing reads are reverse complementary sequences and the 9 nucleotide sequences in the sequencing reads are 5' to adjacent genomic positions.

In some embodiments, the method comprises washing of the barcoded first and second nucleic acids from the planar solid support occurs before the ligating and the ligating occurs in a solution washed from the planar solid support.

In some embodiments, the ligating occurs in a solution on the planar solid support and washing of the barcoded first and second nucleic acids from the planar solid support occurs after the ligating and before the gap filling.

In some embodiments, the method is repeated for a plurality (e.g., at least 3, 5 ,10, 20, 50, 100 or more) beads linked to 5' ends of a plurality of clonal barcoding oligonucleotides, the barcoding oligonucleotides comprising a 5' PCR handle sequence, a 3' capture sequence and a barcode sequence unique to the bead to which the barcode oligonucleotide is linked, and wherein the bridging oligonucleotides comprise (i) a 3' end sequence complementary to the 3' capture sequence of the clonal barcoding oligonucleotides and (ii) a 5' end sequence complementary to the single-stranded 5' portion of the transposase oligonucleotide, thereby determining sequencing reads having barcodes from amplified barcoded barcoding oligonucleotides were from adjacent beads for at least a portion (e.g., at least 5%, 10%, 20%, 40%, 50% or more) of the beads on the tissue section, thereby generating a 2-D map of beads on the tissue section.

In some embodiments, the tagging further comprises tagging nucleic acids in the tissue section such that two or more types of nucleic acids are tagged and subsequently sequenced.

In some embodiments, the two types of nucleic acids are selected from the group consisting of genomic DNA or cDNA.

### BRIEF DESCRIPTION OF THE DRAWINGS

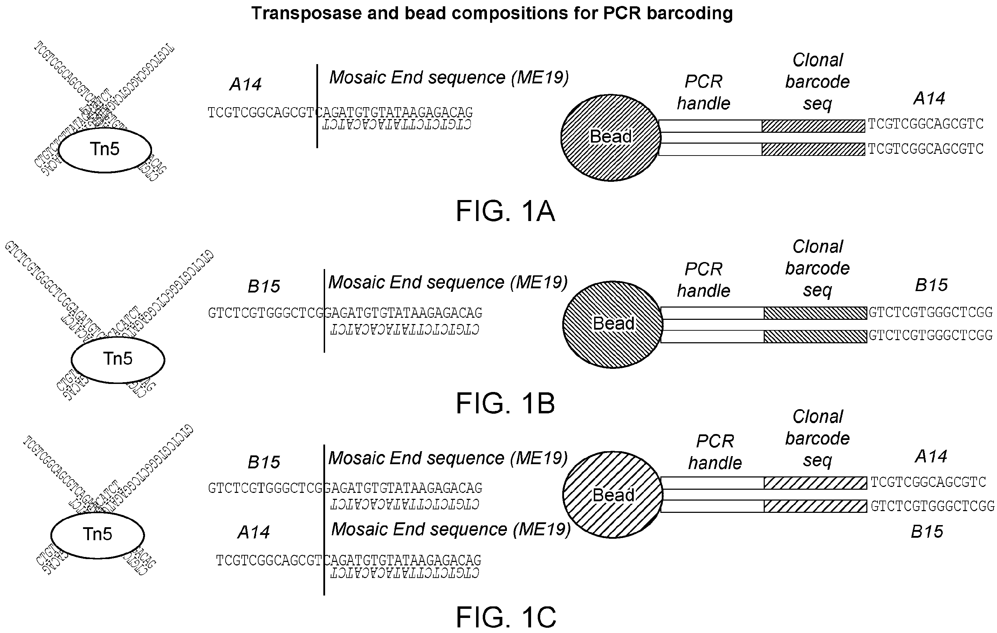

FIG. 1A-C: The transposase, here indicated as Tn5 is pre-loaded with oligonucleotide adapters (transposase oligonucleotides), whereby both adapters contain sequences that match and / or are complementary to the primer binding sequences of the clonal barcode oligonucleotides. In one embodiment as shown in FIG. 1A, the adapters are A14-ME19 homoadapters that contain the A14 sequence that matches the primer binding sequence of the clonal barcode oligo. In FIG. 1B, the adapters are B15-ME19 homoadapters that contain the B15 sequence that matches the primer binding sequence of the clonal barcode oligo. In FIG. 1C, the adapters are both A14-ME19 and B15-ME19, i.e. heteroadapters, as they contain the A14 and B15 sequences that matches both primer binding sequences of a clonal barcode oligonucleotide that has two primer binding sequences. The proportion of the two different barcoding oligonucleotides may be 50:50 but may for example vary (e.g., 1:99 or 99: 1). Although only two barcoding oligonucleotides are shown per bead in this figure, barcoding oligonucleotides per bead can range, for example, from 100 000 to 100 billion or more. The Tn5 adapters can be optionally phosphorylated.

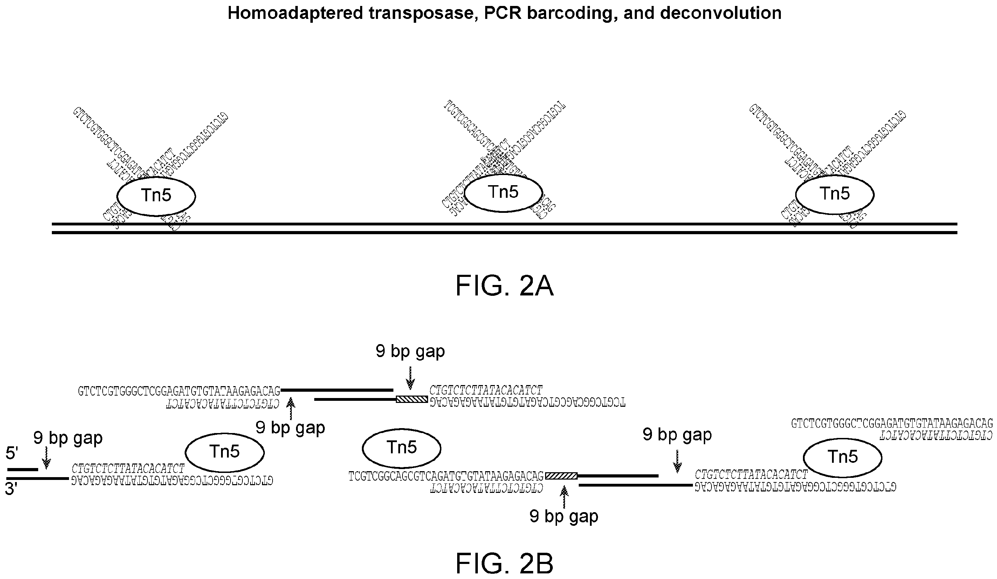

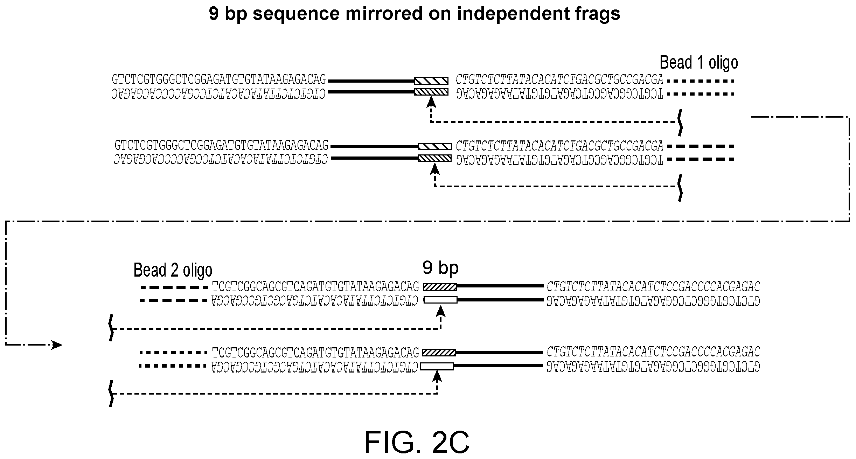

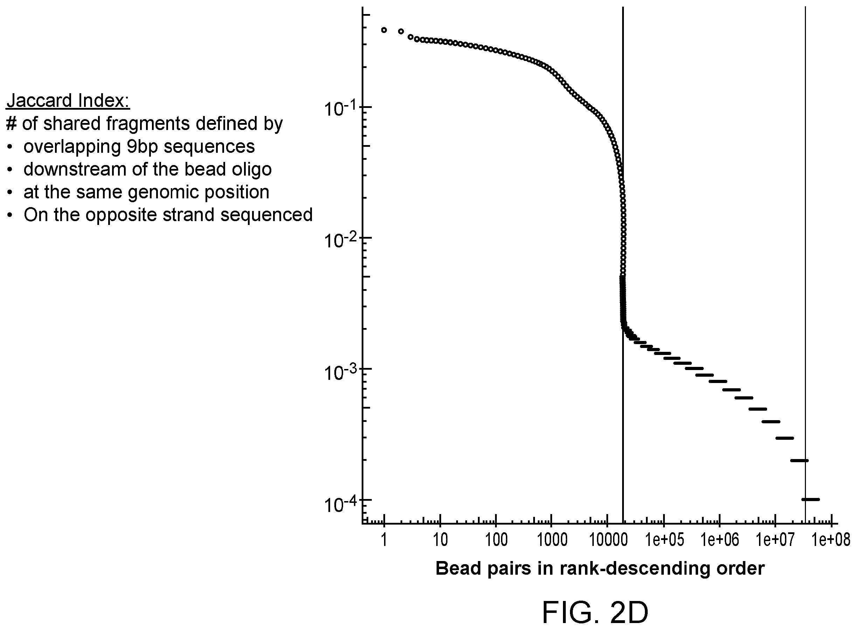

FIG. 2A-D: Homoadaptered Tn5 transposases tagment DNA as shown in FIG. 2A) The products of the tagmentation reaction illustrated in FIG. 2B have 9 bp gaps for each cut site on opposite strands of the molecule. Prior to FIG. 2C, the Tn5 is removed and the gaps are filled and molecules are blunt ended to provide A14 and B15 complements on the opposite strands. PCR then occurs in FIG. 2C using barcoding oligonucleotides from Bead 1 or Bead 2 during different PCR cycles. Bioinformatic analyses providing a jaccard index (FIG. 2D) links oligonucleotides from different beads to a unique tagmentation event at a specific genomic location.

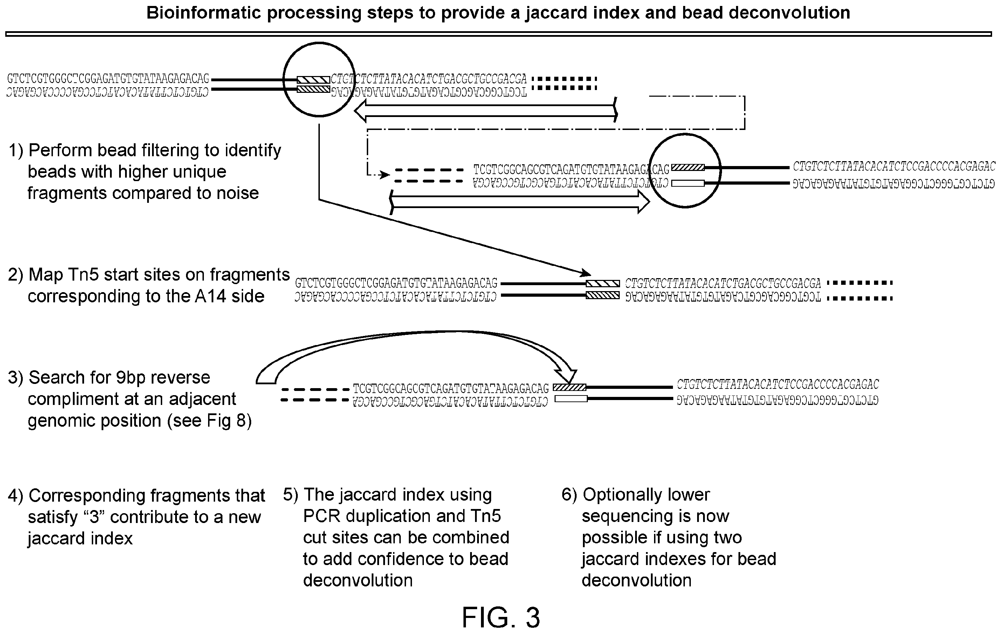

FIG. 3: Bioinformatic processing steps to provide a jaccard index and bead deconvolution.

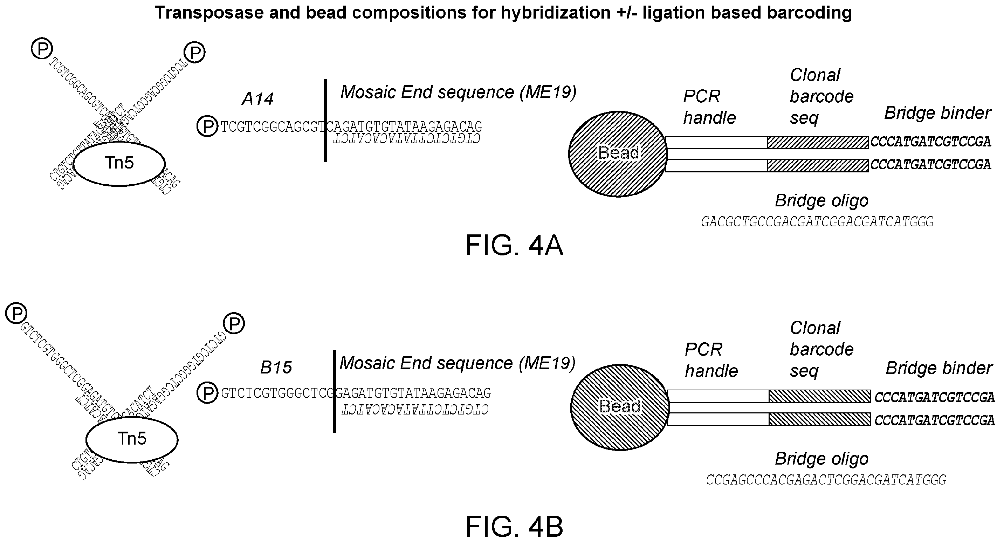

FIG. 4A-B: As shown, the transposase, here indicated as Tn5, can be pre-loaded with oligonucleotide adapters (transposases oligonucleotides), whereby both adapters contain sequences that match and / or are complementary to the bridge oligonucleotide sequence, which are themselves complementary to the terminus of the bead oligonucleotide sequence referred to in this figure as the "bridge oligo." The Tn5-loaded adapters in this figure are all phosphorylated. In one embodiment as shown in FIG. 4A, the adapters are phosphorylated A14-ME19 homoadapters that contain the A14 sequence that matches the bridge oligo sequence of the bridge oligo. In FIG. 4B, the adapters are phosphorylated B15-ME19 homoadapters that contain the B15 sequence that matches the bridge oligo sequence of the bridge oligo.

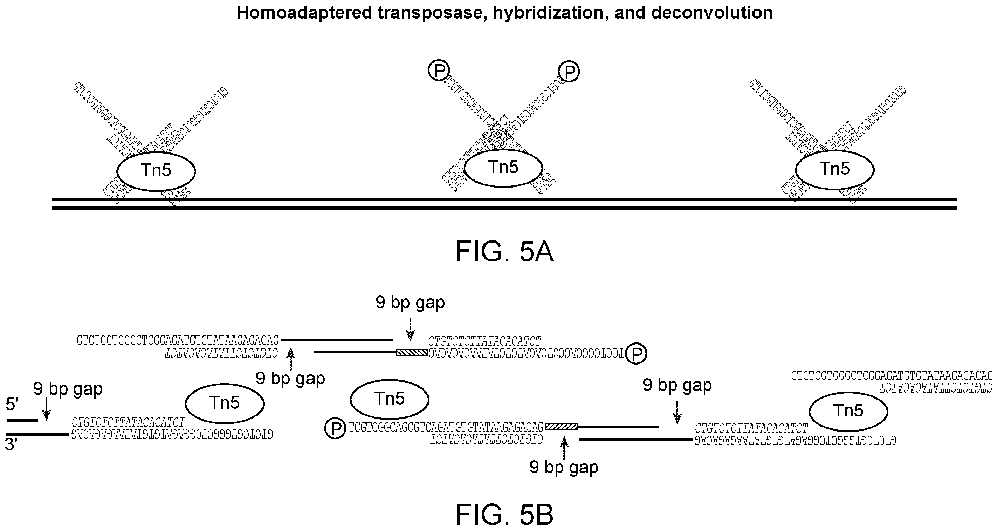

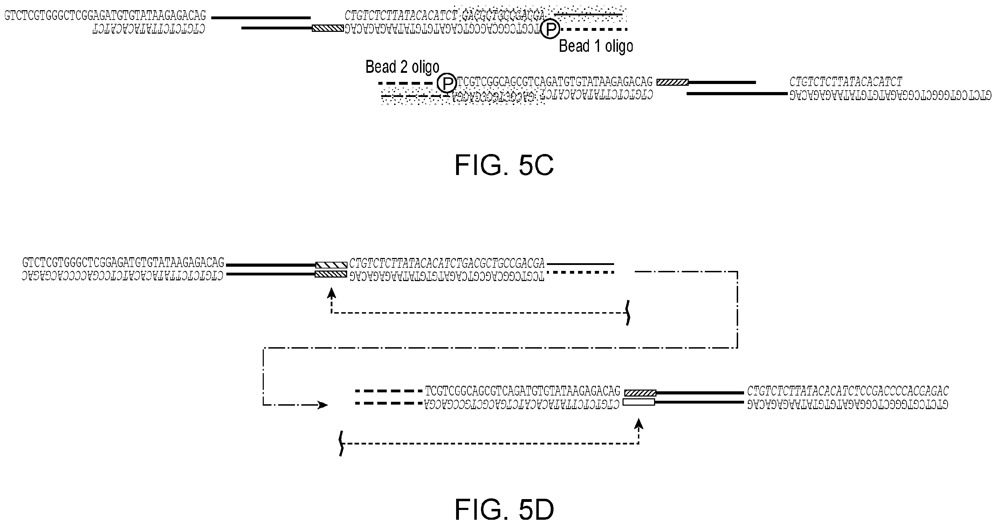

FIG. 5: Barcoding for bead deconvolution occurs through hybridization with or without ligation and not PCR. Homoadaptered Tn5 transposases tagment DNA as shown in FIG. 5A. The products of the tagmentation reaction illustrated in FIG. 5B have 9 bp gaps for each cut site on opposite strands of the molecule. Prior to FIG. 5C, the Tn5 is removed, however the gaps are not filled and the molecules still have sticky ends. Hybridization then occurs in FIG. 5C using oligonucleotides from Bead 1 or Bead 2 and the corresponding bridge. After hybridization, ligation occurs, followed by gap filling and blunt ending the molecules. The dotted line refers to the identification of a shared unique Tn5 transposase across two barcoding oligonucleotides from beads and thus from two beads by the bioinformatic method described in FIGs. 2 and 3.

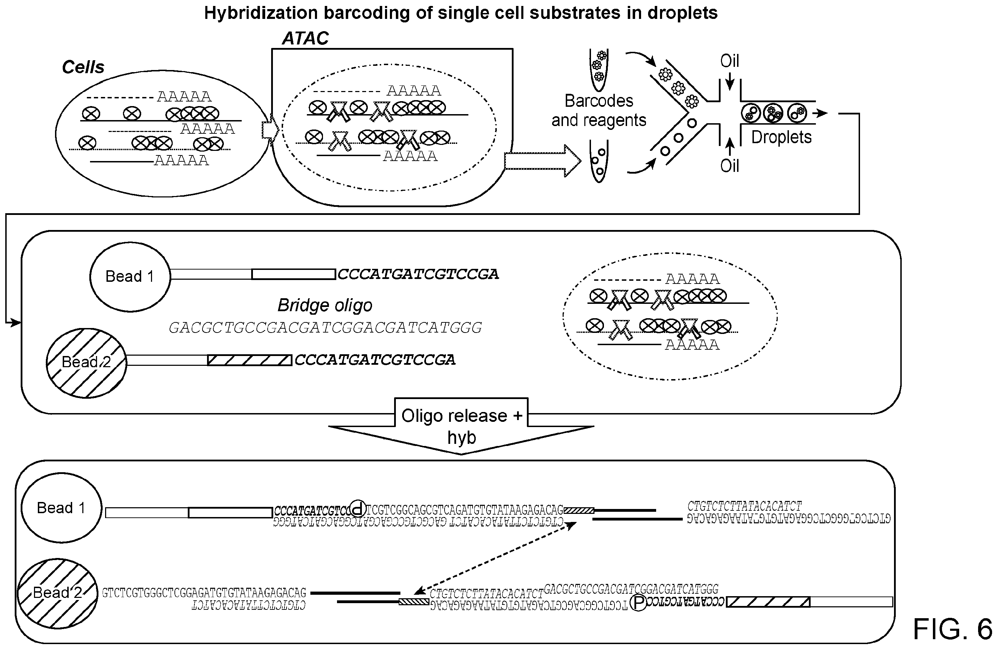

FIG. 6: Hybridization barcoding of single cell substrates in droplets. FIG. 6 depicts hybridization-based single cell barcoding in droplets with bead deconvolution to allow for co-localization of beads to single droplets. Cells and /or nuclei are tagmented with homoadaptered Tn5 transposases. They are then encapsulated together with beads linked to barcoding oligonucleotides and reagents. Once the beads and tagmented cells or nuclei are encapsulated, the oligonucleotides are released and hybridize to bridge oligonucleotides that also hybridize to phosphorylated transposase oligonucleotide adapters. The barcoding oligonucleotides from the beads and phosphorylated transposase oligonucleotides are then ligated downstream (not shown). Comparison of the shared 9bp sequence on opposite sequenced strands shown by a dotted line between the rectangles allows for deconvolution of the beads to the same original droplet. If oligonucleotide release is not enzyme-dependent, hybridization-based barcoding can occur in the presence of a strong protein denaturant (e.g., proteinase K and / or guanidine thiocyanate). Use of such as strong protein denaturant in this barcoding method can in some embodiments increase molecular conversion rates and sensitivity by releasing the substrates to solution.

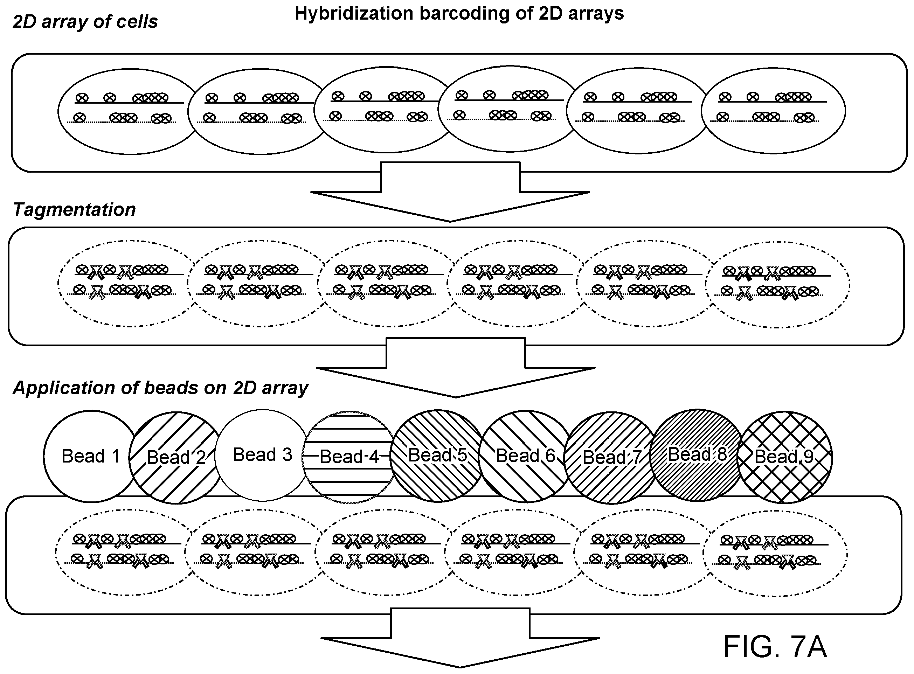

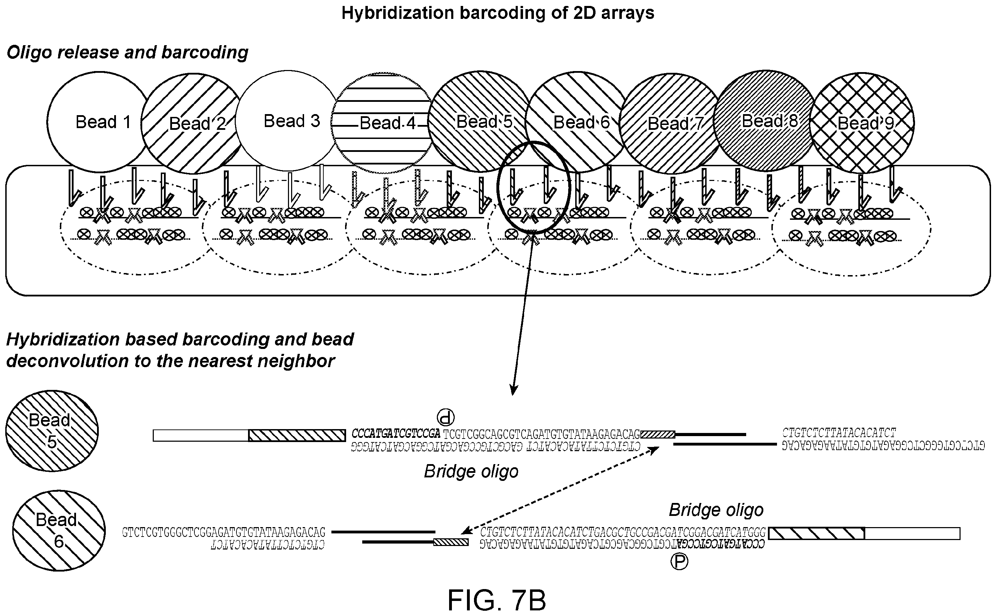

FIG. 7A-B: Hybridization barcoding of 2D arrays. FIGs. 7A-B depict hybridization based spatial barcoding on a 2D array with bead deconvolution to allow for co-localization of beads to their nearest neighbor. In a 2D array, cells and /or nuclei are tagmented with homoadaptered Tn5 transposases. Beads linked to barcoding oligonucleotides are then applied to the 2D array. The oligonucleotides are released and hybridize to bridge oligonucleotides that also hybridize to phosphorylated Tn5 adapters. The bead barcoding oligonucleotides and phosphorylated Tn5 adapter are then ligated downstream (not shown). Comparison of the shared 9 bp sequence on opposite sequenced strands shown by a dotted line between the rectangles allows for deconvolution of the beads to their nearest neighbor. This can be done across the 2D array to reconstruct the location of the beads without a priori knowledge of their locations.

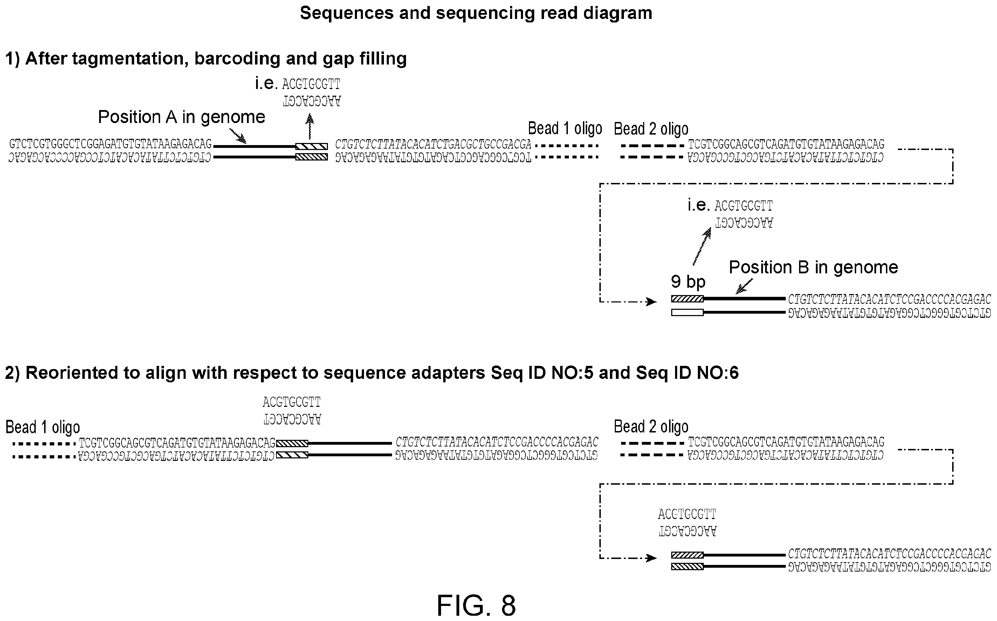

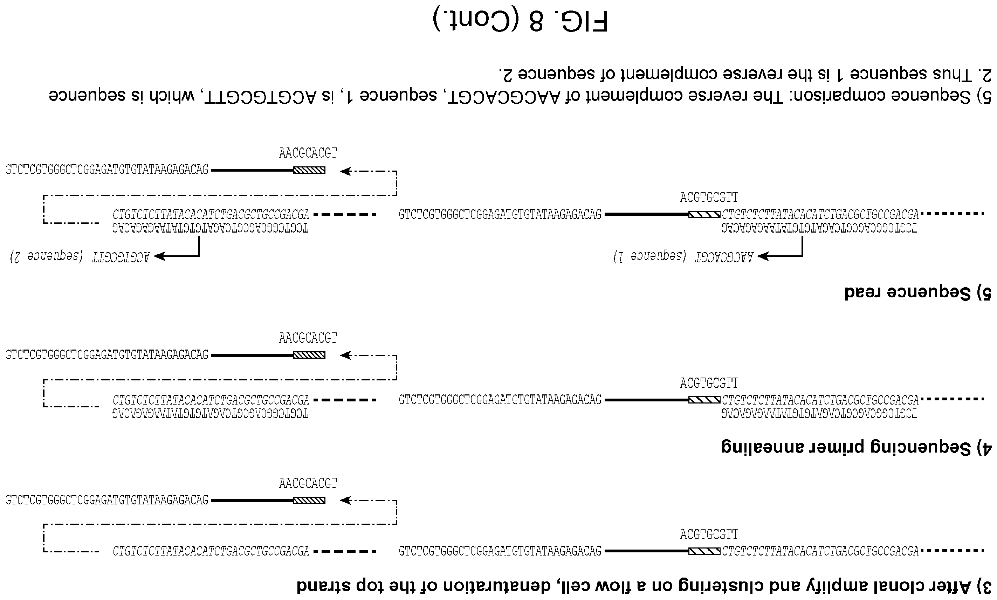

FIG. 8 illustrates one embodiment of generating sequence reads for determining whether the 9 nucleotide sequences are 5' of adjacent sequences (as compared to the genomic or cDNA sequences of the sample being sequenced) and reverse complements.

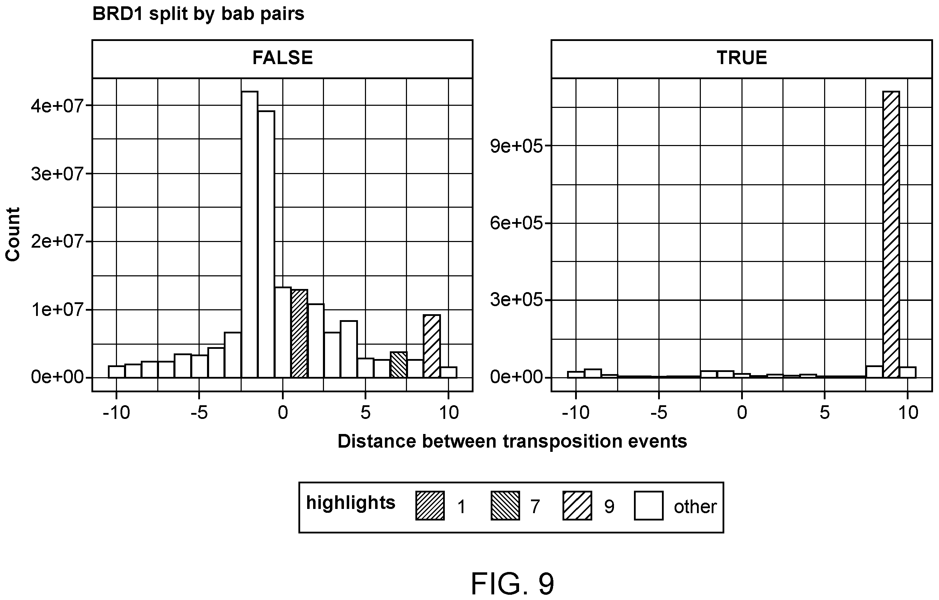

FIG. 9 shows the abundance of metric d (distance between fragments) between adjacent tn5 transposition. Notable distances 1, 7 and 9 are shown in darker bars. Data is split into panels of transposition pairs predicted to be in the same droplet (TRUE) or not in the same droplet (FALSE).

### DEFINITIONS

Unless defined otherwise, all technical and scientific terms used herein generally have the same meaning as commonly understood by one of ordinary skill in the art to which this invention belongs. Generally, the nomenclature used herein and the laboratory procedures in cell culture, molecular genetics, organic chemistry, and nucleic acid chemistry and hybridization described below are those well-known and commonly employed in the art. Standard techniques are used for nucleic acid and peptide synthesis. The techniques and procedures are generally performed according to conventional methods in the art and various general references (see generally, Sambrook et al. MOLECULAR CLONING: A LABORATORY MANUAL, 2d ed. (1989) Cold Spring Harbor Laboratory Press, Cold Spring Harbor, N.Y), which are provided throughout this document. The nomenclature used herein and the laboratory procedures in analytical chemistry, and organic synthetic described below are those well-known and commonly employed in the art.

The term "amplification reaction" refers to any *in vitro* means for multiplying the copies of a target sequence of nucleic acid in a linear or exponential manner. Such methods include two-primer methods such as polymerase chain reaction (PCR); ligase methods such as DNA ligase chain reaction (see US Patents 4,683,195 and 4,683,202; PCR Protocols: A Guide to Methods and Applications (Innis et al., eds, 1990)) (LCR); QBeta RNA replicase and RNA transcription-based amplification reactions (e.g., amplification that involves T7, T3, or SP6 primed RNA polymerization), such as the transcription amplification system (TAS), nucleic acid sequence based amplification (NASBA), and self-sustained sequence replication (3SR); isothermal amplification reactions (e.g., single-primer isothermal amplification (SPIA)); as well as others known to those of skill in the art.

"Amplifying" refers to a step of submitting a solution to conditions sufficient to allow for amplification of a polynucleotide if all of the components of the reaction are intact. Components of an amplification reaction include, e.g., primers, a polynucleotide template, polymerase, and nucleotides. The term "amplifying" typically refers to an "exponential" increase in target nucleic acid. However, "amplifying" as used herein can also refer to linear increases in the numbers of a select target sequence of nucleic acid, such as is obtained with cycle sequencing or linear amplification. In an exemplary embodiment, amplifying refers to PCR amplification using a first and a second amplification primer.

The term "amplification reaction mixture" refers to an aqueous solution comprising the various reagents used to amplify a target nucleic acid. These include enzymes, aqueous buffers, salts, amplification primers, target nucleic acid, and nucleoside triphosphates. Amplification reaction mixtures may also further include stabilizers and other additives to optimize efficiency and specificity. Depending upon the context, the mixture can be either a complete or incomplete amplification reaction mixture.

"Polymerase chain reaction" or "PCR" refers to a method whereby a specific segment or subsequence of a target double-stranded DNA, is amplified in a geometric progression. PCR is well known to those of skill in the art; see, *e.g.,* US Patents 4,683,195 and 4,683,202; and PCR Protocols: A Guide to Methods and Applications, Innis et al., eds, 1990. Exemplary PCR reaction conditions typically comprise either two or three step cycles. Two step cycles have a denaturation step followed by a hybridization/elongation step. Three step cycles comprise a denaturation step followed by a hybridization step followed by a separate elongation step.

A "primer" refers to a polynucleotide sequence that hybridizes to a sequence on a target nucleic acid and serves as a point of initiation of nucleic acid synthesis. Primers can be of a variety of lengths and are often less than 50 nucleotides in length, for example 12-30 nucleotides, in length. The length and sequences of primers for use in PCR can be designed based on principles known to those of skill in the art, see, *e.g.,* Innis *et al*., *supra.* Primers can be DNA, RNA, or a chimera of DNA and RNA portions. In some cases, primers can include one or more modified or non-natural nucleotide bases. In some cases, primers are labeled.

A nucleic acid, or a portion thereof, "hybridizes" to another nucleic acid under conditions such that non-specific hybridization is minimal at a defined temperature in a physiological buffer (*e.g.,* pH 6-9, 25-150 mM chloride salt). In some cases, a nucleic acid, or portion thereof, hybridizes to a conserved sequence shared among a group of target nucleic acids. In some cases, a primer, or portion thereof, can hybridize to a primer binding site if there are at least about 6, 8, 10, 12, 14, 16, or 18 contiguous complementary nucleotides, including "universal" nucleotides that are complementary to more than one nucleotide partner. Alternatively, a primer, or portion thereof, can hybridize to a primer binding site if there are 0, or fewer than 2 or 3 complementarity mismatches over at least about 12, 14, 16, 18, or 20 contiguous nucleotides. In some embodiments, the defined temperature at which specific hybridization occurs is room temperature. In some embodiments, the defined temperature at which specific hybridization occurs is higher than room temperature. In some embodiments, the defined temperature at which specific hybridization occurs is at least about 37, 40, 42, 45, 50, 55, 60, 65, 70, 75, or 80 °C. In some embodiments, the defined temperature at which specific hybridization occurs is 37, 40, 42, 45, 50, 55, 60, 65, 70, 75, or 80 °C.

As used herein, "nucleic acid" means DNA, RNA, single-stranded, double-stranded, or more highly aggregated hybridization motifs, and any chemical modifications thereof. Modifications include those providing chemical groups that incorporate additional charge, polarizability, hydrogen bonding, electrostatic interaction, points of attachment and functionality to the nucleic acid ligand bases or to the nucleic acid ligand as a whole. Such modifications include peptide nucleic acids (PNAs), phosphodiester group modifications (*e.g*., phosphorothioates, methylphosphonates), 2'-position sugar modifications, 5-position pyrimidine modifications, 8-position purine modifications, modifications at exocyclic amines, substitution of 4-thiouridine, substitution of 5-bromo or 5-iodo-uracil; backbone modifications, methylations, unusual base-pairing combinations such as the isobases, isocytidine and isoguanidine. Nucleic acids can also include non-natural bases, such as, for example, nitroindole. Modifications can also include 3' and 5' modifications including capping with a fluorophore (*e.g*., quantum dot) or another moiety.

A "polymerase" refers to an enzyme that performs template-directed synthesis of polynucleotides, *e.g*., DNA and/or RNA. The term encompasses both the full length polypeptide and a domain that has polymerase activity. DNA polymerases are well-known to those skilled in the art, including DNA polymerases isolated or derived from *Pyrococcus furiosus*, *Thermococcus litoralis,* and *Thermotoga maritime,* or modified versions thereof. Additional examples of commercially available polymerase enzymes include: Klenow fragment (New England Biolabs^{®} Inc.), Taq DNA polymerase (QIAGEN), 9 °N^{™} DNA polymerase (New England Biolabs^{®} Inc.), Deep Vent^{™} DNA polymerase (New England Biolabs^{®} Inc.), Manta DNA polymerase (Enzymatics^{®}), *Bst* DNA polymerase (New England Biolabs^{®} Inc.), and phi29 DNA polymerase (New England Biolabs^{®} Inc.).

Polymerases include both DNA-dependent polymerases and RNA-dependent polymerases such as reverse transcriptase. At least five families of DNA-dependent DNA polymerases are known, although most fall into families A, B and C. Other types of DNA polymerases include phage polymerases. Similarly, RNA polymerases typically include eukaryotic RNA polymerases I, II, and III, and bacterial RNA polymerases as well as phage and viral polymerases. RNA polymerases can be DNA-dependent and RNA-dependent.

As used herein, the term "partitioning" or "partitioned" refers to separating a sample into a plurality of portions, or "partitions." Partitions are generally physical, such that a sample in one partition does not, or does not substantially, mix with a sample in an adjacent partition. Partitions can be solid or fluid. In some embodiments, a partition is a solid partition, *e.g*., a microchannel or microwell. In some embodiments, a partition is a fluid partition, *e.g*., a droplet. In some embodiments, a fluid partition (*e.g.,* a droplet) is a mixture of immiscible fluids (*e.g.,* water and oil). In some embodiments, a fluid partition (*e.g*., a droplet) is an aqueous droplet that is surrounded by an immiscible carrier fluid (*e.g.,* oil).

In some cases partitions are virtual. In a preferred embodiment, virtual partitions require a physical alteration of a molecule or group of molecules, wherein the alteration identifies a unique partition for that molecule or group of molecules. Typical physical alterations suitable for establishing or maintaining virtual partitioning include, without limitation, nucleic acid barcodes, and detectable labels. Cell fixation and / or embedding cells in hydrogel particles may be required to enable the physical alterations. For example, a sample can be physically partitioned in a hydrogel, and the components of each partition tagged with a partition-specific identifier (*e.g.,* a nucleic acid barcode sequence) such that the identifier is unique as compared to other partitions but shared between the components of the partition. The partition-specific identifier can then be used to maintain a virtual partition in downstream applications that involve combining of the physically partitioned material. Thus, if the sample is a sample of cells physically partitioned into partitions containing a single cell, the identifier can identify different nucleic acids that derived from a single cell after partitions are recombined.

As used herein, a "tag" refers to a non-target nucleic acid component, generally DNA, that provides a means of addressing a nucleic acid fragment to which it is joined. For example, in preferred embodiments, a tag comprises a nucleotide sequence that permits identification, recognition, and/or molecular or biochemical manipulation of the DNA to which the tag is attached (*e.g.*, by providing a unique or partition-specific sequence, and/or a site for annealing an oligonucleotide, such as a primer for extension by a DNA polymerase, or an oligonucleotide for capture or for a ligation reaction). The process of joining the tag to the DNA molecule is sometimes referred to herein as "tagging" and DNA that undergoes tagging or that contains a tag is referred to as "tagged" (*e.g.,* "tagged DNA")." A tag can be a barcode, an adapter sequence, a primer hybridization site, or a combination thereof.

The term "bead" refers to any solid support that can be in a partition, e.g., a small particle or other solid support. In some embodiments, the beads comprise polyacrylamide. For example, in some embodiments, the beads incorporate barcode oligonucleotides into the gel matrix through an acrydite chemical modification attached to each oligonucleotide. Exemplary beads can also be hydrogel beads. In some cases, the hydrogel is in *sol* form. In some cases, the hydrogel is in *gel* form. An exemplary hydrogel is an agarose hydrogel. Other hydrogels include those described in, *e.g.,* US Patents 4,438,258; 6,534,083; 8,008,476; 8,329,763; US 2002/0009591 A1; US 2013/0022569 A1; US 2013/0034592 A1; WO 1997/030092; and WO 2001/049240.

Methods of linking oligonucleotides to beads are described in, e.g., WO 2015/200541. In some embodiments, the oligonucleotide configured to link the hydrogel to the barcode is covalently linked to the hydrogel. Numerous methods for covalently linking an oligonucleotide to one or more hydrogel matrices are known in the art. As but one example, aldehyde derivatized agarose can be covalently linked to a 5'-amine group of a synthetic oligonucleotide. In some embodiments, the forward primers are linked to the bead or solid support via a cleavable linker (as described below) and can be cleaved from the bead or solid support in the partitions. In some embodiments, a second oligonucleotide primer that functions as a reverse primer in combination with the first oligonucleotide primer on a target nucleic acid can be included in the partitions, or alternatively following combining of partitions into a bulk reaction. The target reverse primer, for example, will include a sequence that hybridizes to a reverse complement sequence on the target under the conditions of the assay to allow, for example, for polymerase-based extension.

As used herein a "barcode" is a short nucleotide sequence (*e.g*., about 1, 2, 3, 4, 5, 6, 7, 8, 9, 10, 11, 12, 15, 20, 25 or more nucleotides long) that identifies a molecule to which it is conjugated. Barcodes can be used, for example, to identify molecules in a reaction mixture or partition. Generally, a partition-specific barcode should be unique for that partition as compared to barcodes present in other partitions. For example, partitions containing target RNA from single-cells can be subject to reverse transcription conditions using primers that contain a different partition-specific barcode sequence in each partition, thus incorporating a copy of a unique "cellular barcode" into the reverse transcribed nucleic acids of each partition. Thus, nucleic acids from each cell can be distinguished from nucleic acid of other cells due to the presence of the unique "cellular barcode." In some cases, the cellular barcode is provided as a "bead barcode" that is present on oligonucleotides conjugated to a particle or bead (*e.g.,* a magnetic bead), wherein the bead barcode is shared by (*e.g*., identical or substantially identical amongst) all, or substantially all, of the oligonucleotides conjugated to that bead. Thus, cellular and bead barcodes can be present in a partition, attached to a bead, or bound to cellular nucleic acid as multiple copies of the same barcode sequence. Cellular or bead barcodes of the same sequence can be identified as deriving from the same cell, partition, or bead. Such partition-specific, cellular, or bead barcodes can be generated using a variety of methods, which methods can result in the barcode conjugated to or incorporated into a solid or hydrogel support (*e.g.,* a solid bead or particle or hydrogel bead or particle). In some cases, the partition-specific, cellular or bead barcode is generated using a split and mix (also referred to as split and pool) synthetic scheme. A partition-specific barcode can be a cellular barcode and/or a bead barcode. Similarly, a cellular barcode can be a partition-specific barcode and/or a bead barcode. Additionally, a bead barcode can be a cellular barcode and/or a partition-specific barcode. As described more herein, in some embodiments, at least some partitions receive, and thus contain, two or more beads, resulting in two or more bead-specific barcodes in one partition. The present disclosure addresses, in part, how to decipher this.

In other cases, barcodes uniquely identify the molecule to which it is conjugated. For example, by performing reverse transcription or PCR amplification using primers that each contain a "unique molecular identifier" barcode. In still other examples, primers can be utilized that contain "partition-specific barcodes" unique to each partition, and "molecular barcodes" unique to each molecule. After barcoding, partitions can then be combined, and optionally amplified, while maintaining virtual partitioning. Thus, *e.g.,* the presence or absence of a target nucleic acid (*e.g.,* reverse transcribed nucleic acid) comprising each barcode can be counted (*e.g.* by sequencing) without the necessity of maintaining physical partitions. In some cases, the unique molecular identifier barcode is encoded by a contiguous sequence of nucleotides tagged to one end of a target nucleic acid.

In some cases, the unique molecular identifier barcode is encoded by a non-contiguous sequence. Non-contiguous UMIs can have a portion of the barcode at a first end of the target nucleic acid and a portion of the barcode at a second end of the target nucleic acid. In some cases, the UMI is a non-contiguous barcode containing a variable length barcode sequence at a first end and a second identifier sequence at a second end of the target nucleic acid. In some cases, the UMI is a non-contiguous barcode having a variable length barcode sequence at a first end and a second identifier sequence at a second end of the target nucleic acid, wherein the second identifier sequence is determined by a position of a transposase fragmentation event, *e.g.,* a transposase fragmentation site and transposon end insertion event.

The length of the barcode sequence can determine how many unique samples can be differentiated. For example, a 1 nucleotide barcode can differentiate 4, or fewer, different samples or molecules; a 4 nucleotide barcode can differentiate 4⁴ or 256 samples or less; a 6 nucleotide barcode can differentiate 4096 different samples or less; and an 8 nucleotide barcode can index 65,536 different samples or less. Additionally, barcodes can be attached to both strands of a target nucleic acid molecule (*e.g*., gDNA or cDNA) either through barcoded primers for both first and second strand synthesis, through ligation, or in a tagmentation reaction.

A "transposase" or "tagmentase" means an enzyme that is capable of forming a functional complex with a transposon end-containing composition and catalyzing insertion or transposition of the transposon end-containing composition into the double-stranded target DNA with which it is incubated in an *in vitro* transposition reaction. Typically, the insertion or transposition results in fragmentation of the target DNA.

A "transposase" or "tagmentase" means an enzyme that is capable of forming a functional complex with a transposon end-containing composition and catalyzing insertion or transposition of the transposon end-containing composition into the double-stranded target DNA with which it is incubated in an *in vitro* transposition reaction. Typically, the insertion or transposition results in fragmentation of the target DNA.

The term "transposon end" means a double-stranded DNA that contains or consists of the nucleotide sequences (the "transposon end sequences") that are necessary to form the complex with the transposase that is functional in an *in vitro* transposition reaction. A transposon end forms a "complex" or a "synaptic complex" or a "transposome complex" or a "transposome composition" with a transposase or integrase that recognizes and binds to the transposon end, and which complex is capable of inserting or transposing the transposon end into target DNA with which it is incubated in an *in vitro* transposition reaction. A transposon end exhibits two complementary sequences consisting of a "transferred transposon end sequence" or "transferred strand" and a "non-transferred transposon end sequence," or "non-transferred strand" For example, one transposon end that forms a complex with a hyperactive Tn5 transposase (*e.g*., EZ-Tn5^{™} Transposase, EPICENTRE Biotechnologies, Madison, Wis., USA) that is active in an *in vitro* transposition reaction comprises a transferred strand that exhibits a "transferred transposon end sequence" as follows:
5' AGATGTGTATAAGAGACAG 3' (SEQ ID NO:4),
and a non-transferred strand that exhibits a "non-transferred transposon end sequence" as follows:
5' CTGTCTCTTATACACATCT 3' (SEQ ID NO: 7).

The 3'-end of a transferred strand is joined or transferred to target DNA in an *in vitro* transposition reaction. The non-transferred strand, which exhibits a transposon end sequence that is complementary to the transferred transposon end sequence, is not joined or transferred to the target DNA in an *in vitro* transposition reaction.

In another example, a transposon end that forms a complex with a transposase that is active in an *in vitro* transposition reaction comprises a transferred strand that exhibits a "transferred transposon end sequence" as follows:
5' -TCGTCGGCAGCGTCAGATGTGTATAAGAGACAG - 3' (SEQ ID NO:5); or
5' - GTCTCGTGGGCTCGGAGATGTGTATAAGAGACAG -3 (SEQ ID NO:6).
and a non-transferred strand that exhibits a "non-transferred transposon end sequence" as follows:
5' CTGTCTCTTATACACATCT 3' (SEQ ID NO:7).

In some embodiments, a transposon end-containing composition comprises a transferred transposon end and a non-transferred transposon end that form a double-stranded nucleotide composition. In some embodiments, a transposon end comprises a double-stranded nucleotide composition having a nucleotide sequence necessary to form a functional complex with a transposase resulting in insertion of the transposon ends into one or more of the target nucleic acid molecules with which it is incubated in an *in vitro* transposition reaction. In some embodiments, the double-stranded nucleotide composition corresponding to the transposon end comprises from 5' to 3' AGATGTGTATAAGAGACAG (SEQ ID NO 4) and from 5' to 3' CTGTCTCTTATACACATCT (SEQ ID NO:7). In another embodiment, the double-stranded nucleotide composition corresponding to the transposon end comprises from 5' to 3' TCGTCGGCAGCGTCAGATGTGTATAAGAGACAG (SEQ ID NO:5) and from 5' to 3' CTGTCTCTTATACACATCT (SEQ ID NO:7). In yet another embodiment, the double-stranded nucleotide composition corresponding to the transposon end comprises from 5' to 3' GTCTCGTGGGCTCGGAGATGTGTATAAGAGACAG (SEQ ID NO:6) and from 5' to 3' CTGTCTCTTATACACATCT (SEQ ID NO:7).

Whether "the 9 nucleotide sequences in sequencing reads are 5' to adjacent genomic positions" refers to performing sequencing based on primers from the same primer hybridization sequence introduced by the transposase oligonucleotide, resulting in sequences reads, which if aligned with genomic DNA show that the two reads in question are from adjacent sequences in the genome (or cDNA) and therefore are "adjacent" and that in the sequencing reads the 9 nucleotide sequence is 5' from the target nucleic acid sequence. This is illustrated, for example, in FIG. 8. Two fragments are "adjacent" because they were formed from a cleavage event and thus when mapped back to a genome they align to adjacent sequences. As noted elsewhere herein, the cleavage event caused by a transposase results in the "top" strand of one fragment having the 9 nucleotide sequence and the "bottom" strand of the second fragment having the reverse complement of the 9 nucleotide sequence.

### DETAILED DESCRIPTION OF THE INVENTION

Tagmentation is a process commonly used to fragment DNA to be sequenced while simultaneously adding known oligonucleotide sequences delivered by a transposase to the end of the so-created fragments. Tagmentation works via transposition of a transposase, e.g., Tn5 or a variant thereof. Tn5 performs a "cut and paste" function, in which the Tn5 inserts into a target sequence, creating a 9-bp duplication of the target (see, e.g., Reznikoff WS. Transposon Tn5. *Annu. Rev. Genet.* **42**:269-86 (2008)). This results in a 9 bp gap between the nontransferred strand from the transposase oligonucleotide and the target DNA. Thus, the transposition results in a cleavage site in the target DNA, resulting in a first and second DNA fragment, wherein the two fragments have a complementary 9 nucleotide sequence.

In partition-based nucleotide sequencing sample preparation, partitions (e.g., droplets) contain target DNA and also contain one or more beads carrying bead-specific barcodes for barcoding the target DNA in the partition. When only one bead is in a partition, all target DNA in the partition is barcoded with the same barcode and when contents of partitions are later combined in a sequencing workflow, one can track back that DNA tagged with that bead's barcode were all within one partition. However, where two or more beads are introduced into a partition (e.g., as a function of Poisson distributions) different DNA fragments from one partition will receive different barcodes (from different beads). If different bead barcodes are interpreted in sequencing reads as being from different partitions, this can create issues with sequencing accuracy.

The inventors have discovered a method of using the 9-base pair sequence, which is found on two fragments formed from a cleavage site caused by transposition, to determine when two beads were in the same partition, allowing one to consolidate sequencing reads having different bead barcodes but coming from the same partition. For example, the inventors have found that sequencing reads having different barcodes are nevertheless from the same partition if, between two DNA fragments demonstrate sequences indicating the sequenced fragments from formed by the same cleavage event yet have different barcodes. For example this can be determined for sequencing reads having barcodes two different barcoding oligonucleotides can be determined to be from the same partition if the 9 nucleotide sequences (resulting from the transposase cleavage) in the sequencing reads are reverse complementary sequences and the 9 nucleotide sequences in the sequencing reads are 5' to adjacent genomic positions. This aspect can be used advantageously in a number of ways. In some embodiments, sequencing reads from a plurality of barcodes can be allocated to a specific partition even if the sequencing reads contain different partition-specific (e.g., bead) barcodes if they meet the above-described criteria. In other embodiments, there is only one partition-specific barcode sequence in the partition, and this method is used to confirm that there is not a second partition-specific barcode sequence.

In view of this discovery, DNA samples can be prepared in new and improved ways to take advantage of this finding. For example, in some embodiments as detailed below, the sample preparation workflow can involve only hybridization reactions within partitions, allowing one to avoid, if desired, enzymatic manipulation of the sample in the partitions. This can be especially beneficial in situations in which it is desirable to treat the partitions under conditions (e.g., high temperature, the presence of chaotropic or other enzyme-harming and / or digestion agents) that would otherwise harm enzymes in partitions.

Moreover, this discovery also has applications in spatial profiling, for example for providing gene expression or sequencing information about fixed tissue samples in the context of spatial location in the fixed sample. As described in more detail below, this can involve contacting permeabilized tissue that contain DNA that has been fragmented by tagmentation with beads comprising oligonucleotide barcodes that are then used to barcode the fragments in the tissue. This may involve releasing the oligonucleotides from the beads to enable contact with the nucleic acid substrates (i.e., the target nucleic acid fragments in cells in the tissue).Adjacent beads with different barcodes can barcode fragments from the same tagmentation cleavage site, resulting in a situation analogous to having multiple beads in a partition as described above. Harvested barcoded DNA from the tissue can be sequenced and the location of adjacent beads in the sample can be determined based on the different barcodes tagged to fragments originating from the same cleavage site. For example, if sequencing reads having different barcodes are from adjacent beads, the sequence identity of the 9 nucleotide sequence will be the same, the 9 nucleotide sequences (resulting from the transposase cleavage) in the sequencing reads will be reverse complementary sequences and the 9 nucleotide sequences in the sequencing reads will be 5' to adjacent genomic positions. Thus, location of adjacent beads can be compiled by detecting this situation across a plurality of beads, allowing one to prepare a map of different barcodes, allowing one to ascribe a relative location to sequencing reads on the permeabilized fixed tissue.

In some embodiments, the method comprises partitioning a sample comprising one or more target nucleic acids within cells or nuclei into a plurality of partitions. In some embodiments, the sample comprising target nucleic acids comprises DNA, RNA, or a combination or hybrid thereof. In some embodiments, the sample comprises target nucleic acids situated in single cell or single nuclei. In some embodiments, intact cells or nuclei can be permeabilized to allow entry of reagents. Exemplary reagents can include the use of digitonin, or fixatives such as methanol, or paraformaldehyde. In some embodiments, the sample comprises target nucleic acids that are isolated from tissue or cells. In some embodiments, the cells will have intact chromatin such that some chromosomal regions are more accessible to the transposase than other chromosomal regions, allowing for ATACseq results to be generated. In some embodiments, the DNA will be stripped of histones prior to transposition allowing for genotyping results to be generated. One method to remove histones is by using lithium 3,5-diiodosalicylic acid as described in Lithium-assisted nucleosome depletion (LAND). *See, e.g.,* Vitak et al., Nat Methods. 2017 Mar; 14(3): 302-308. Another method is to cross link the cells using formaldehyde followed by quenching with glycine and application of SDS (*see, e.g.,* Mulqueen et al., Nat Biotechnol. 2018 Jun; 36(5): 428-431). In some embodiments, NaOH is used on tissue paraffin embedded tissue samples after digestion with pepsin as described in PCR in Situ Hybridization (MSP-ISH) approaches (see, e.g., Nuovo et al., Proc Natl Acad Sci USA 96: 12754-12759).

In some embodiments, the sample comprising target nucleic acids is a biological sample. Biological samples can be obtained from any biological organism, *e.g*., an animal, plant, fungus, pathogen (e.g., bacteria or virus), or any other organism. In some embodiments, the biological sample is from an animal, *e.g.,* a mammal (*e.g.,* a human or a non-human primate, a cow, horse, pig, sheep, cat, dog, mouse, or rat), a bird (*e.g*., chicken), or a fish. A biological sample can be any tissue or bodily fluid obtained from the biological organism, *e.g*., blood, a blood fraction, or a blood product (*e.g*., serum, plasma, platelets, and red blood cells), sputum or saliva, tissue (*e.g*., kidney, lung, liver, heart, brain, nervous tissue, thyroid, eye, skeletal muscle, cartilage, or bone tissue); cultured cells, *e.g.*, primary cultures, explants, and transformed cells, stem cells, stool, and urine. In some embodiments, the sample is a sample comprising cells. In some embodiments, the sample is a single-cell sample.

In some embodiments prior to introduction into partitions and prior to transposition, the RNA in cells in the tissue can be converted to cDNA *in situ.* For examples, cells or nuclei can be fixed and the RNA can reverse transcribed by adding the appropriate reverse transcription regents (e.g., a reverse transcriptase, nucleotides, one or more primer, which optionally is a primer comprising a polyT 3' end) to form first strand cDNA molecules. As a further possible step, the first strand cDNA can be converted to double stranded cDNA through second strand synthesis (e.g., by providing appropriate reagents, e.g., an appropriate primer and DNA polymerase).

In some embodiments, prior to introduction into partitions, DNA (e.g., chromosomal DNA, cDNA, or other DNA) in the cells or nucleic will be tagmented to cleave the DNA into fragments that receive at their ends oligonucleotides delivered by the tagmentation transposase ("transposase oligonucleotides"). The cells can be permeabilized and the nuclear DNA within can be fragmented, for example with a tranposase that introduces adapter sequences to the ends of the fragmented DNA. Where isolated nuclei are used, the nuclei need not be permeabilized for entry to the transposase into the nuclei. The action of the transposase sometimes referred to as "tagmentation" and can involve introduction of different transposase oligonucleotides on different sides of a DNA breakage point or the transposase oligonucleotides added can be identical. Homoadapter-loaded tagmentases are tagmentases that contain transposase oligonucleotides of only one sequence, which transposase oligonucleotide is added to both ends of a tagmentase-induced breakpoint in the genomic DNA. Heteroadapter-loaded tagmentases are tagmentases that contain two different transposase oligonucleotides, such that a different transposase oligonucleotide sequence is added to the two DNA ends created by a tagmentase-induced breakpoint in the DNA. These two different transposase oligonucleotides may be different at only a portion of their sequence, i.e. between SEQ ID NO:5 and SEQ ID NO:6. Adapter loaded tagmentases are further described, e.g., in US 2010/0120098 A1; US 2012/0301925 A1; US 2015/0291942 A1 and US Patents 5,965,443; 6,437,109; 7,083,980; 9,005,935; and 9,238,671. In ATAC-seq applications, quantifying the number of intact sequencing reads (indicating heterochromatin not cleaved by transposase) one can measure chromatin structure.

Transposase oligonucleotides are partially double-stranded and partially single-stranded. The single-stranded portion typically is a 5' single stranded overhang sequence that is optionally 5' phosphorylated and that optionally comprises a universal sequence that allows for interaction with the barcode oligonucleotides. Interaction with the barcode oligonucleotides can involve hybridization to a bridging oligonucleotide, which in turn hybridizes to the barcode oligonucleotides. Alternatively, interaction with the barcode oligonucleotides can comprise using the barcode oligonucleotides as a template for the synthesis of a complement of the universal sequence, the complement of which is used as a primer binding site during primer extension DNA synthesis in downstream molecular biology reactions. These DNA fragments post transposition may be covalently linked through the use of ligases. Optionally, the transposase oligonucleotide can also include for example a second barcode sequence, such as a unique molecular identifier sequence and / or a sample index. The second barcode sequence can be for example 4-10 base pairs long. While the single-stranded portion typically is a 5' single stranded overhang sequence, in some embodiments, instead the single-stranded portion is a 3' single stranded overhang sequence.

A tagmentase is an enzyme that is capable of forming a functional complex with a transposon end-containing composition and catalyzing insertion or transposition of the transposon end-containing composition into the double-stranded target DNA with which it is incubated in an *in vitro* transposition reaction. Exemplary transposases include modified Tn5 transposases that are hyperactive compared to wildtype Tn5, for example can have one or more mutations selected from E54K, M56A, or L372P. Wild-type Tn5 transposon is a composite transposon in which two near-identical insertion sequences (IS50L and IS50R) are flanking three antibiotic resistance genes (Reznikoff WS. Annu Rev Genet 42: 269-286 (2008)). Each IS50 contains two inverted 19-bp end sequences (ESs), an outside end (OE) and an inside end (IE). However, wild-type ESs have a relatively low activity and were replaced in vitro by hyperactive mosaic end (ME) sequences. A complex of the transposase with the 19-bp ME is thus all that is necessary for transposition to occur, provided that the intervening DNA is long enough to bring two of these sequences close together to form an active Tn5 transposase homodimer (Reznikoff WS., Mol Microbiol 47: 1199-1206 (2003)). Transposition is a very infrequent event in vivo, and hyperactive mutants were historically derived by introducing three missense mutations in the 476 residues of the Tn5 protein (E54K, M56A, L372P), which is encoded by IS50R (Goryshin IY, Reznikoff WS. 1998. J Biol Chem 273: 7367-7374 (1998)). Transposition works through a "cut-and-paste" mechanism, where the Tn5 excises itself from the donor DNA and inserts into a target sequence, creating a 9-bp duplication of the target (Schaller H. Cold Spring Harb Symp Quant Biol 43: 401-408 (1979); Reznikoff WS., Annu Rev Genet 42: 269-286 (2008)). In current commercial solutions (Nextera^{™} DNA kits, Illumina), free synthetic ME adapters are end-joined to the 5'-end of the target DNA by the transposase (tagmentase). In some embodiments, the tagmentase is linked to a solid support (e.g., a bead that is different from the bead linked to the forward primer). An example commercial bead-linked tagmentase is Nextera^{™} DNA Flex (Illumina).

In some embodiments, the transposase oligonucleotide(s) (also referred to as adapter(s)) is at least 19 nucleotides in length, e.g., 19-100 nucleotides. In some embodiments, the 5' overhang sequence of transposase oligonucleotides is different between heteroadapters, while the double stranded portion (typically 19 bp) is the same. In some embodiments, a transposase oligonucleotide comprises TCGTCGGCAGCGTC (SEQ ID NO:1) or GTCTCGTGGGCTCGG (SEQ ID NO:2). In some embodiments involving the heteroadapter-loaded tagmentase, the tagmentase is loaded with a first transposase oligonucleotide comprising TCGTCGGCAGCGTC (SEQ ID NO:1) and a second transposase oligonucleotide comprising GTCTCGTGGGCTCGG (SEQ ID NO:2). In some embodiments, the transposase oligonucleotide comprises AGATGTGTATAAGAGACAG (SEQ ID NO:3) and the complement thereof (this is the mosaic end and this is the only specifically required cis active sequence for Tn5 transposition). In some embodiments, the transposase oligonucleotide comprises TCGTCGGCAGCGTCAGATGTGTATAAGAGACAG (SEQ ID NO:4) with the complement for AGATGTGTATAAGAGACAG (SEQ ID NO:3) or GTCTCGTGGGCTCGGAGATGTGTATAAGAGACAG (SEQ ID NO:5) with the complement for AGATGTGTATAAGAGACAG (SEQ ID NO:3). In some embodiments involving the heteroadapter-loaded tagmentase, the tagmentase is loaded with a first transposase oligonucleotide comprising TCGTCGGCAGCGTCAGATGTGTATAAGAGACAG (SEQ ID NO:4) with the complement for AGATGTGTATAAGAGACAG (SEQ ID NO:3) and GTCTCGTGGGCTCGGAGATGTGTATAAGAGACAG (SEQ ID NO:5) with the complement for AGATGTGTATAAGAGACAG (SEQ ID NO:3).

Tagmentation of the DNA in the sample forms a series of cleavage sites in the DNA. For convenience, one cleavage site is discussed below but it will be understood the reaction occurs a large number of times. Tagmentation generates at least one cleavage site in a target nucleic acid from one of the cells to form a first nucleic acid fragment and a second nucleic acid fragment, wherein the first and second nucleic acid fragments comprise at the cleavage site a single-stranded 9 nucleotide sequence originating from the target nucleic acid, which are complementary to each other, linked to a transposase oligonucleotide delivered by a tagmentation transposase, wherein the transposase oligonucleotide has a double-stranded portion, and a single-stranded 5' portion. The 9 nucleotide sequence is from the target DNA, with each fragment receiving one strand of the 9 nucleotide sequence. Accordingly, the first and second strands have complementary 9 nucleotide sequences at the cleavage site. Linked to the 9 nucleotide single-stranded sequences is the 3' end of the strand of the transposase oligonucleotide that is double-stranded such that the end of the fragments comprise the double stranded portion of the transposase oligonucleotide and at its other end the single stranded 5' portion of the transposase oligonucleotide.

Following tagmentation, in some embodiments a plurality of partitions are formed from the cells or nuclei containing the tagmented DNA and a plurality of barcode oligonucleotide-linked beads. As discussed more below, the partitions in some embodiments will also include copies of a bridging oligonucleotide.

Methods and compositions for partitioning are described, for example, in WO 2010/036352, US 2010/0173394 A1, US 2011/0092373 A1, and US 2011/0092376 A1. The plurality of partitions can be in a plurality of emulsion droplets, or a plurality of microwells.

In some embodiments, one or more reagents are added during droplet formation or to the droplets after the droplets are formed. Methods and compositions for delivering reagents to one or more partitions include microfluidic methods as known in the art; droplet or microcapsule combining, coalescing, fusing, bursting, or degrading (e.g., as described in US 2015/0027,892 A1; US 2014/0227,684 A1; WO 2012/149,042; and WO 2014/028,537); droplet injection methods (e.g., as described in WO 2010/151,776); and combinations thereof.

As described herein, the partitions can be picowells, nanowells, or microwells. The partitions can be pico-, nano-, or micro- reaction chambers, such as pico, nano, or microcapsules. The partitions can be pico-, nano-, or micro- channels. The partitions can be droplets, *e.g.,* emulsion droplets. In some embodiments, a droplet comprises an emulsion composition, *i.e.,* a mixture of immiscible fluids (*e.g*., water and oil). In some embodiments, a droplet is an aqueous droplet that is surrounded by an immiscible carrier fluid (*e.g.,* oil). In some embodiments, a droplet is an oil droplet that is surrounded by an immiscible carrier fluid (*e.g.,* an aqueous solution). In some embodiments, the droplets described herein are relatively stable and have minimal coalescence between two or more droplets. In some embodiments, less than 0.0001%, 0.0005%, 0.001%, 0.005%, 0.01%, 0.05%, 0.1%, 0.5%, 1%, 2%, 3%, 4%, 5%, 6%, 7%, 8%, 9%, or 10% of droplets generated from a sample coalesce with other droplets. The emulsions can also have limited flocculation, a process by which the dispersed phase comes out of suspension in flakes. In some cases, such stability or minimal coalescence is maintained for up to 4, 6, 8, 10, 12, 24, or 48 hours or more (*e.g.,* at room temperature, or at about 0, 2, 4, 6, 8, 10, or 12 °C). In some embodiments, the droplet is formed by flowing an oil phase through an aqueous sample or reagents.

The oil phase can comprise a fluorinated base oil which can additionally be stabilized by combination with a fluorinated surfactant such as a perfluorinated polyether. In some embodiments, the base oil comprises one or more of a HFE 7500, FC-40, FC-43, FC-70, or another common fluorinated oil. In some embodiments, the oil phase comprises an anionic fluorosurfactant. In some embodiments, the anionic fluorosurfactant is Ammonium Krytox (Krytox-AS), the ammonium salt of Krytox FSH, or a morpholino derivative of Krytox FSH. Krytox-AS can be present at a concentration of about 0.1%, 0.2%, 0.3%, 0.4%, 0.5%, 0.6%, 0.7%, 0.8%, 0.9%, 1.0%, 2.0%, 3.0%, or 4.0% (w/w). In some embodiments, the concentration of Krytox-AS is about 1.8%. In some embodiments, the concentration of Krytox-AS is about 1.62%. Morpholino derivative of Krytox FSH can be present at a concentration of about 0.1%, 0.2%, 0.3%, 0.4%, 0.5%, 0.6%, 0.7%, 0.8%, 0.9%, 1.0%, 2.0%, 3.0%, or 4.0% (w/w). In some embodiments, the concentration of morpholino derivative of Krytox FSH is about 1.8%. In some embodiments, the concentration of morpholino derivative of Krytox FSH is about 1.62%.

In some embodiments, the oil phase further comprises an additive for tuning the oil properties, such as vapor pressure, viscosity, or surface tension. Non-limiting examples include perfluorooctanol and 1H,1H,2H,2H-Perfluorodecanol. In some embodiments, 1H,1H,2H,2H-Perfluorodecanol is added to a concentration of about 0.05%, 0.06%, 0.07%, 0.08%, 0.09%, 0.1%, 0.2%, 0.3%, 0.4%, 0.5%, 0.6%, 0.7%, 0.8%, 0.9%, 1.0%, 1.25%, 1.50%, 1.75%, 2.0%, 2.25%, 2.5%, 2.75%, or 3.0% (w/w). In some embodiments, 1H,1H,2H,2H-Perfluorodecanol is added to a concentration of about 0.18% (w/w).

In some embodiments, the emulsion can be substantially monodisperse. In other embodiments, the emulsion can be polydisperse. Emulsion dispersity can arise from the method of emulsion formation. For example, microfluidic emulsion formation is typically low polydispersity compared to "salad shaker" emulsion formation, which can be highly polydisperse. Polydispersity can also arise downstream of emulsion formation, such as when droplets of the emulsion fuse together.

In some embodiments, the emulsion is formulated to produce highly monodisperse droplets having a liquid-like interfacial film that can be converted by heating into microcapsules having a solid-like interfacial film; such microcapsules can behave as bioreactors able to retain their contents through an incubation period. The conversion to microcapsule form can occur upon heating. For example, such conversion can occur at a temperature of greater than about 40, 50, 60, 70, 80, 90, or 95 °C. During the heating process, a fluid or mineral oil overlay can be used to prevent evaporation. Excess continuous phase oil can be removed prior to heating, or left in place. The microcapsules can be resistant to coalescence and/or flocculation across a wide range of thermal and mechanical processing.

Following conversion of droplets into microcapsules, the microcapsules can be stored at about -70, -20, 0, 3, 4, 5, 6, 7, 8, 9, 10, 15, 20, 25, 30, 35, or 40 °C. In some embodiments, these capsules are useful for storage or transport of partition mixtures. For example, samples can be collected at one location, partitioned into droplets containing enzymes, buffers, and/or primers or other probes, optionally one or more polymerization reactions can be performed, the partitions can then be heated to perform microencapsulation, and the microcapsules can be stored or transported for further analysis.

In some embodiments, the sample is partitioned into, or into at least, 500 partitions, 1000 partitions, 2000 partitions, 3000 partitions, 4000 partitions, 5000 partitions, 6000 partitions, 7000 partitions, 8000 partitions, 10,000 partitions, 15,000 partitions, 20,000 partitions, 30,000 partitions, 40,000 partitions, 50,000 partitions, 60,000 partitions, 70,000 partitions, 80,000 partitions, 90,000 partitions, 100,000 partitions, 200,000 partitions, 300,000 partitions, 400,000 partitions, 500,000 partitions, 600,000 partitions, 700,000 partitions, 800,000 partitions, 900,000 partitions, 1,000,000 partitions, 2,000,000 partitions, 3,000,000 partitions, 4,000,000 partitions, 5,000,000 partitions, 10,000,000 partitions, 20,000,000 partitions, 30,000,000 partitions, 40,000,000 partitions, 50,000,000 partitions, 60,000,000 partitions, 70,000,000 partitions, 80,000,000 partitions, 90,000,000 partitions, 100,000,000 partitions, 150,000,000 partitions, or 200,000,000 partitions.

In some embodiments, the droplets that are generated are substantially uniform in shape and/or size. For example, in some embodiments, the droplets are substantially uniform in average diameter. In some embodiments, the droplets that are generated have an average diameter of about 0.001 µm, about 0.005 µm, about 0.01 µm, about 0.05 µm, about 0.1 µm, about 0.5 µm, about 1 µm, about 5 µm, about 10 µm, about 20 µm, about 30 µm, about 40 µm, about 50 µm, about 60 µm, about 70 µm, about 80 µm, about 90 µm, about 100 µm, about 150 µm, about 200 µm, about 300 µm, about 400 µm, about 500 µm, about 600 µm, about 700 µm, about 800 µm, about 900 µm, or about 1000 µm. In some embodiments, the droplets that are generated have an average diameter of less than about 1000 µm, less than about 900 µm, less than about 800 µm, less than about 700 µm, less than about 600 µm, less than about 500 µm, less than about 400 µm, less than about 300 µm, less than about 200 µm, less than about 100 µm, less than about 50 µm, or less than about 25 µm. In some embodiments, the droplets that are generated are non-uniform in shape and/or size.

In some embodiments, the droplets that are generated are substantially uniform in volume. For example, the standard deviation of droplet volume can be less than about 1 pl (picoliter), 5 pl, 10 pl, 100 pl, 1 nl, or less than about 10 nl. In some cases, the standard deviation of droplet volume can be less than about 10-25% of the average droplet volume. In some embodiments, the droplets that are generated have an average volume of about 0.001 nl, about 0.005 nl, about 0.01 nl, about 0.02 nl, about 0.03 nl, about 0.04 nl, about 0.05 nl, about 0.06 nl, about 0.07 nl, about 0.08 nl, about 0.09 nl, about 0.1 nl, about 0.2 nl, about 0.3 nl, about 0.4 nl, about 0.5 nl, about 0.6 nl, about 0.7 nl, about 0.8 nl, about 0.9 nl, about 1 nl, about 1.5 nl, about 2 nl, about 2.5 nl, about 3 nl, about 3.5 nl, about 4 nl, about 4.5 nl, about 5 nl, about 5.5 nl, about 6 nl, about 6.5 nl, about 7 nl, about 7.5 nl, about 8 nl, about 8.5 nl, about 9 nl, about 9.5 nl, about 10 nl, about 11 nl, about 12 nl, about 13 nl, about 14 nl, about 15 nl, about 16 nl, about 17 nl, about 18 nl, about 19 nl, about 20 nl, about 25 nl, about 30 nl, about 35 nl, about 40 nl, about 45 nl, or about 50 nl.

As noted above, the partitions will contain a single cells or nuclei and one or more sets of a plurality of clonal barcoding oligonucleotides, the barcoding oligonucleotides comprising a 5' PCR handle sequence, a 3' capture sequence and a barcode sequence unique to the set. In most embodiments described herein, the clonal barcoding oligonucleotides are delivered to the partitions linked to beads, which conveniently deliver a set of clonal oligonucleotides to the partition, and thus the barcodes therein indicate the bead to which the barcode oligonucleotide is linked. However, in every instance as described herein where a "bead" is described for providing the set of clonal barcoding oligonucleotides, it should be appreciated that the set could alternatively be delivered to the partitions in droplets, each of which contain a different set of clonal barcodes, such that the barcode is unique to the droplet that contains the set. In these cases, the droplets carrying the clonal barcoding oligonucleotides can be merged into the partitions and in some embodiments more than one droplet is merged into the partition resulting in different barcoding oligonucleotides having different barcode sequences introduced into a partition.

In embodiments in which the transposase delivers two different oligonucleotides (e.g., hetero adaptors) the barcoding oligonucleotides on the beads may be a mixture of two different oligonucleotides, some having one 5' PCR handle sequence and some having a different PCR handle sequence to accommodate the two heteroadaptor oligonucleotides delivered by the transposase. In some embodiments, the proportion of the two different 5' PCR handle sequence may be 50:50 but alternatively they can be any ratio, for example 1:99 or 99: 1. In some embodiments, a mixture of two different transposases containing different homoadapters that are unique per tranposase delivers two different oligonucleotides. In these embodiments, the barcoding oligonucleotides on the beads may be of only a single sequence and specific to one of the two different homoadaptered transposases. Oligos used in PCR downstream will be specific to the other homoadaptered transposase adapter.

The 3' capture sequence of the barcoding oligonucleotide will vary depending on which embodiment of the workflow is employed. In a first embodiment (depicted for example in FIG. 1-2), the 3' capture sequence comprises the universal sequence in the single-stranded 5' portion of the transposase oligonucleotide, allowing for the 3' capture sequence to capture the tagmented fragment following a gap filling step. In this embodiment, the single-stranded 5' portions of the tagmented fragments are filled in with a polymerase to generate a fully double-stranded fragment. Thus, the 9 nucleotide sequence as well as the single-stranded portion of the transposase oligonucleotide linked the fragments is filled in, the latter creating a reverse complement sequence of the single-stranded 5' portion of the transposase oligonucleotide. The reverse complement sequence of the universal sequence will be complementary to the 3' capture sequence of the barcoding oligonucleotide, allowing for linkage via hybridization and primer extension synthesis of the barcoding oligonucleotide and the tagmented DNA fragment.

Alternatively, the partitions can further contain a bridging oligonucleotide that forms a bridge via hybridization between the tagmented target fragment and the barcoding oligonucleotide. In this embodiment, the bridging oligonucleotides comprise (i) a 3' end sequence complementary to the 3' capture sequence of the clonal barcoding oligonucleotides and (ii) a 5' end sequence complementary to the universal sequence of the single-stranded 5' portion of the transposase oligonucleotide, allowing the bridging oligonucleotide to hybridize on one side to the clonal barcoding oligonucleotide and on the other side to the transposase oligonucleotide on the fragmented cell DNA. See, e.g., FIG. 4A-B.

In either option described above (with our without the bridging oligonucleotide), a gap filling step occurs to fill in any single-stranded sequences with their complementary sequence on the other strand. For example, in all cases, the 9-nucleotide single-stranded sequences on the target nucleic acid fragments are gap-filled to make the 9-base pair sequences double-stranded. In addition, the 5' single-stranded overhang sequence from the transposases oligonucleotide will also be rendered double-stranded by gap-filling. Where bridging oligonucleotide-enabled hybridization occurs, downstream gap-filling may also include synthesizing the complement of all or a part of the barcode oligonucleotide to create a primer binding site for downstream PCR. In these embodiments, gap-filling may occur in partitions or downstream in bulk (after partition contents have been combined). Gap-filling occurs by introducing a suitable polymerase and nucleotides under conditions to allow the polymerase to fill in single-stranded gaps in the sequence. Exemplary gap filling polymerases can include, for example, T4 DA polymerase of other DNA polymerase I enzymes. Nicks remaining following gap filling can be ligated (e.g., with T4 DNA ligase) to remove the nicks.

In embodiments where the bridging oligonucleotides are not employed, gap-filling prior to hybridization so that the complement of the universal sequence on the 5' single-stranded overhang of the attached transposases oligonucleotide can be formed, which as explained above is subsequently hybridized to the 3' capture sequence of the barcoding oligonucleotide. Thus in these embodiments, gap-filling occurs in the partitions.

Once the fragments have been tagged with barcodes, the contents of the partitions can be combined into a bulk solution such that the remaining steps involve treatment of a single bulk solution containing the contents of the various partitions. In some embodiments, partitions are broken by mixing the partitions (e.g., droplets) with a destabilizing fluid. In some embodiments, the destabilizing fluid is chloroform. In some embodiments, the destabilizing fluid comprises a perfluorinated alcohol. In some embodiments, the destabilizing fluid comprises a fluorinated oil, such as a perfluorocarbon oil. In some embodiments, the partitions are microwells and the barcoded products are retrieved from microwells by removing the bead containing immobilized oligonucleotides. In some embodiments, the barcoded products are retrieved from microwells by retrieving the released barcode oligonucleotides attached to the target nucleic acid fragments.

In embodiments employing the bridge oligonucleotides, gap-filling occurs after hybridization, allowing the gap-filling to occur after the contents of partitions are combined in bulk. One advantage of this aspect is that the partitions themselves need not include any enzymes, allowing for inclusion of reagents in the partitions that would otherwise harm enzymes. For example, in some embodiments, it can be desirable to include (i) one or more proteases (for example proteinase K) to eliminate protein that may be present, (ii) surfactants (e.g., ionic surfactants, e.g., SDS and nonioinic surfactants, e.g., NP-40), or (iii) a chaotropic agent (for example guanidine thiocyanate or KOH). Using protein denaturants and / or removal agents allows for the maximal access of nucleic acids to be tagged and barcoded by the barcode oligonucleotides thus improving the overall molecular conversion rate and sensitivity of molecular barcoding.

After the bridge oligonucleotide is hybridized to the universal sequence in the 5' single-stranded overhang from the transposase oligonucleotide and the 3' capture sequence of the barcoding oligonucleotide, these sequences are ligated with a ligase. The ligation step can occur in the partitions, or following combining the contents of the partitions in bulk under conditions that retain hybridization of the oligonucleotides as described above. Any suitable ligase can be used, either introduced into the partitions or into the bulk mixture as appropriate. In these embodiments, after ligation, gap-filling occurs to fill in the 9-base pair sequences and synthesize a complement of the barcode oligo including universal primer sequences that are used downstream during PCR.

Regardless whether a bridging oligonucleotide is used or not, the methods described form barcoded first and second nucleic acid fragments for each cleavage site caused by the transposases in the initial transposase reaction. Following a standard Poisson distribution some partitions comprising beads linked to barcoding oligonucleotides will comprise at least two beads, meaning two different barcoding oligonucleotides will be in one partition, resulting in some fragments (e.g., a first nucleic acid and a second nucleic acid) formed from a single cleavage site to receive different barcoding oligonucleotides. In other words, the first fragment will be linked to a first barcoding oligonucleotide from a first bead and a second fragment will be linked to a second barcoding oligonucleotide from a second bead. As described below, this occurrence can be detected by detecting the same 9-nucleotide sequence that are on two fragments from the same cleavage event even though the fragments contain different barcoding oligonucleotides. By recognizing that two fragments resulted from a single cleavage event, and identifying the different barcoding oligonucleotides that tagged those two fragments, one can then bin all sequencing reads having both barcodes (the first barcode and the second barcode in the example above) as being from the same partition, allowing one to interpret all of the sequencing reads as being from the same partition even though two barcoding beads were present in the partition. This inference can be extended to a third or fourth or additional different barcoding oligonucleotides in one partition by detection of different single fragmentation events that have different pairs of barcoding oligonucleotides linked to them. For example, if barcoding beads A, B, and C were present in a partition, this can be detected by detecting a first pair of fragments barcoded with A and B, a second pair of fragments barcodes with B and C, and optionally a third pair of fragments with A and C. Pairs of fragments are identified as pairs from a single transposon cleavage event in view of the presence of the same 9 nucleotide sequence at adjacent genetic locations.

Following generation of the barcoded first and second nucleic acid fragments for each cleavage site (which as noted above can occur with or without the bridging oligonucleotide), the resulting tagged first and second nucleic acid fragments can be amplified, e.g., using PCR, for example with primers directed to primer binding sequences in the tagged sequences, For example, PCR handle sequences can be introduced in as part of the forward primers described herein and these PCR handle sequences can be hybridized to by primers to amplify the barcoded first and second nucleic acid fragments. As shown in FIG. 1, PCR handle sequences can conveniently be those sequences that allow one to use primers standard in Illumina-based sequencing, i.e., PCR handle sequences that are complementary to A14 or B15 primer sequences. In one embodiment, as shown in FIG. 1A, the adapters are A14-ME19 homoadapters that contain the A14 sequence that matches the primer binding sequence of the clonal barcode oligonucleotide. In FIG. 1B, the adapters are B15-ME19 homoadapters that contain the B15 sequence that matches the primer binding sequence of the clonal barcode oligonucleotide. In FIG. 1C, the adapters are both A14-ME19 and B15-ME19, i.e. heteroadapters, as they contain the A14 and B15 sequences that matches both primer binding sequences of a clonal barcode oligonucleotide that has two primer binding sequences. Note while the barcoded "first and second" nucleic acid fragments are discussed herein it should be appreciated that this will happen in parallel for all fragments formed from cleavage by the transposon and prepared as described herein.

The resulting amplicons can then be sequenced by any nucleotide sequencing technology desired. Methods for high throughput sequencing and genotyping are known in the art. For example, such sequencing technologies include pyrosequencing, sequencing-by-ligation, single molecule sequencing, sequence-by-synthesis (SBS), massive parallel clonal, massive parallel single molecule SBS, massive parallel single molecule real-time, and massive parallel single molecule real-time nanopore technology. Morozova and Marra provide a review of some such technologies in Genomics, 92: 255 (2008).

Exemplary DNA sequencing techniques include fluorescence-based sequencing methodologies (*See,* e.g., Birren et al., Genome Analysis: Analyzing DNA, 1, Cold Spring Harbor, N.Y). In some embodiments, automated sequencing techniques understood in that art are utilized. In some embodiments, the present technology provides parallel sequencing of partitioned amplicons (PCT Publication No. WO 2006/0841,32). In some embodiments, DNA sequencing is achieved by parallel oligonucleotide extension (s*ee*, *e.g.,* US Patents 5,750,341; and 6,306,597). Additional examples of sequencing techniques include the Church polony technology (Mitra et al., 2003, Analytical Biochemistry 320, 55-65; Shendure et al., 2005 Science 309, 1728-1732; and US Patents 6,432,360; 6,485,944; 6,511,803), the 454 picotiter pyrosequencing technology (Margulies et al., 2005 Nature 437, 376-380; US 2005/0130173 A1), the Solexa single base addition technology (Bennett et al., 2005, Pharmacogenomics, 6, 373-382; US Patents 6,787,308; and 6,833,246), the Lynx massively parallel signature sequencing technology (Brenner et al. (2000). Nat. Biotechnol. 18:630-634; U.S. Pat. Nos. 5,695,934; 5,714,330), and the Adessi PCR colony technology (Adessi et al. (2000). Nucleic Acid Res. 28, E87; WO 2000/018957).

Sequencing reads will include at least part of the original nucleic acid sample fragment sequence, including the 9 bp region, and the barcode introduced by the barcoding oligonucleotide. From these sequencing reads, the 9 bp region can be identified, for example as being adjacent to the oligonucleotide sequence introduced by the transposase (the transposase oligonucleotide). Moreover, the nucleic acid sample fragment comprising the 9 bp region as well as the region downstream of the 9 bp region can also be mapped to a source sequence using any appropriate sequence database (e.g., Genbank) allowing for identification of the nucleic acid sample fragment within a database genomic or cDNA sequence. Different nucleic acid sample fragment reads can be mapped to the same sequence database, allowing one to reveal the nucleic acid sample fragments that are adjacent in the genomic DNA or a cDNA of the cell. Sequencing reads having barcodes from different (e.g., a first and second) barcoding oligonucleotides are considered to be from the same partition if the 9 nucleotide sequences in the sequencing reads are reverse complementary sequences and the 9 nucleotide sequences in the sequencing reads are 5' to adjacent genomic positions (i.e., mapped in the sequence data base to adjacent genomic or cDNA sequences, indicating they are likely from the same cleavage event).

Alignment can be performed by a variety of algorithms. Algorithms can include BLAST and BLAST 2.0 algorithms, which are described in Altschul et al. (1990) J. Mol. Biol. 215: 403-410 and Altschul et al. (1977) Nucleic Acids Res. 25: 3389-3402, respectively. Software for performing BLAST analyses is publicly available through the National Center for Biotechnology Information (NCBI) web site. Other options include BLAT (Kent, Genome Res., 2002 Apr;12(4):656-64), and SOAP (Li et al., Bioinformatics, Volume 24, Issue 5, 1 March 2008, Pages 713-714).

Sequencing reads having barcodes from different (e.g., a first and second) barcoding oligonucleotides that are considered to be from the same partition enables the inference that the beads from which these oligonucleotides originated were located in the same partition during the barcoding reaction. Upon co-localization of the barcode oligonucleotides, the data attributed to each barcode can be merged in silico allowing for an intact data set for the target nucleic acids that were originally contained within the partition.

As well as their use in high-throughput sequencing, the methods described herein can be used for spatial profiling. Spatial profiling is a method for highly multiplex spatial profiling of proteins or RNAs suitable for use on formalin-fixed, paraffin-embedded (FFPE) samples (see, e.g., Beecham, Methods Mol Biol. 2055:563-583 (2020)). As explained in Beecham, "this method uses small photocleavable oligonucleotide "barcodes" (PC-oligos) covalently attached to in-situ affinity reagents (antibodies and RNA-probes) to provide unlimited multiplexing capability. The photocleavage light is projected onto the tissue slice using two-digital micromirror devices (DMD), containing one-million semiconductor-based micromirrors allowing complete flexibility in the pattern of light utilized for high-plex digital profiling of the tissue." See also, Merritt, et al., Nature Biotechnology volume 38, pages586-599 (2020).

The methods described herein allow to improved spatial profiling methods by using *in situ* tagmentation in a fixed (e.g., FFPE) tissue sample. Following tagmentation of nucleic acids with transposase oligonucleotides as described herein, the tissue can be contacted with beads linked to clonal barcoding oligonucleotides and bridging oligonucleotides as described above. Alternatively, the tissue can be contacted with released barcoding oligonucleotides from beads in near proximity to the tissue as well as bridging oligonucleotides. Fragments caused by tagmentation will cause generation of nucleic acid fragments (referred to as a first and second nucleic acid fragment herein, though it will be appreciated this will occur many times in a cell or tissue). In some situations, the first nucleic acid fragment will be tagged with a barcoding oligonucleotide from a first bead and the second nucleic acid fragment will be tagged with a barcoding oligonucleotide from a second (adjacent) bead. Just as two barcoding oligonucleotides being associated with one cleavage event can be used to trace two different barcoding oligonucleotides to the same partition in the examples above, in the context of a fixed tissue and spatial profiling one can trace two different barcoding oligonucleotides as being from adjacent locations in the tissue (and thus allowing barcoding oligonucleotides to be in close proximity such that two fragments from one cleavage site receive different clonal barcodes). While this method is described here in the context of a single cleavage site and two barcodes and associated beads, the method can be repeated a many times allowing for the development of a map of different beads on the tissue where different adjacent beads are identified in relation to each other by this method. In addition to locating the beads, the remaining clonal barcoding beads can be used to tag nucleic acids in the tissue, allowing for any variety of genetic sequences to be sequenced at the same time, providing both position and genetic sequencing information traced to the barcoding oligonucleotide. In tagging various other nucleic acid substrates, this may expand the application space to include other modalities, including RNA, DNA, nucleosome positioning, methylation, and / or 3D configuration. The deconvolution information to co-localize beads using the transposase cleavage position methods described here can be applied to any other nucleic acid that has been tagged (barcoded) even though deconvolution information is not available from those other substrates per se.

In the spatial profiling methods, the tagged nucleic acid fragments can be washed from the tissue section before or after a ligation step. The ligating step ligates the clonal barcodes to the nucleic acid fragments to which they are indirectly hybridized via a bridging oligonucleotide, thereby forming barcoded first and second nucleic acids. Thus ligation can occur *in situ* on the tissue section or in a bulk solution that has been washed from the tissue section and containing the tagged nucleic acids. If the ligation occurs *in situ* the resulting ligation products are then washed from the tissue section.

Remaining single-stranded portion of the tagged nucleic acids, including for example, the 9 nucleotide sequence, can be gap-filled as described above, wherein the gap filling comprises using a polymerase to insert nucleotides using the single stranded sequences as a template. The resulting product can then be amplified (e.g., via PCR) similar to as descried above, using one or more primer, for example a primer that hybridizes to the PCR handle sequences incorporate with the clonal barcoding oligonucleotides.

Sequencing reads can be generated from the amplified barcoded first and second nucleic acid fragments, as described above, wherein the sequencing reads include the barcode sequence, the 9 nucleotide sequence and at least a portion of the nucleic acid fragment from the tissue. Using alignment, one can identify in the sequence reads the genomic location relative to the nucleic acid fragment and sequence identity of the 9 nucleotide duplication sequence. One can then determine sequencing reads having barcodes from the amplified barcoded first and second barcoding oligonucleotides were from adjacent beads on the tissue section if the 9 nucleotide sequences in the sequencing reads are reverse complementary sequences and the 9 nucleotide sequences in the sequencing reads are 5' to adjacent genomic positions. Methods for determining this can comprise the steps as displayed in FIG. 8.

As noted above, for spatial profiling the above method can take place many times in parallel thereby generating a linkage map of different beads based on identification of relatively rare events in which adjacent beads supply different barcoding oligonucleotides to different fragments from the same cleavage event. This information can be used to generate a map of the beads, which optionally can be overlaid with other information resulting from the same beads, for example genotype or nucleic acid sequence frequency information as generated from other sequencing reads from the same beads using nucleic acids in that location of the tissue sample as described herein.

### EXAMPLES

### Example 1 (Prophetic):

50,000 nuclei are tagmented with transposases in an Eppendorf tube. Due to contiguity preservation by the transposase the nuclei remain as intact units. The nuclei are then encapsulated into droplets together with barcoding reagents, i.e, beads linked to barcode oligonucleotides, hybridization buffer, as well as guanidine thiocyanate. Guanidine thiocyanate will denature the proteins and release the maximal amount of transposase adapter ends for barcoding. The oligonucleotides are released from the bead and hybridize, through the use of a bridge oligonucleotide, the transposase adapter is ligated to genomic DNA. After hybridization, the droplets are broken, the DNA collected on ampure beads, the guanidine thiocyanate is removed by washing, and the tagged DNA substrates are released into master mixes that support ligation to covalently link the barcoding oligonucleotide to ATAC fragment generated by the transposases. This is followed by gap filling and PCR enrichment. The barcoded fragments are then sequenced. After sequencing, a bioinformatic pipeline is launched to perform the following steps for bead deconvolution: 1) Beads are filtered to identify beads with higher unique fragments compared to background; 2) transposase start sites on fragments downstream of barcode sequences are mapped; 3) All fragments are compared with each other to identify reverse complements of the first 9 bp followed by the adjacent genomic region. The data are pooled together to generate a jaccard index, whereby union is defined by shared overlapping 9bp reverse complement sequences at adjacent genomic locations. Higher than noise jaccard indexes between beads are used to co-localize beads to the same droplet. This information is used to de-fractionate single cell data.

### Example 2:

60,000 nuclei were tagmented with transposases in an Eppendorf tube. As in the prophetic example above, due to contiguity preservation by the transposase, the nuclei remained as intact units. The nuclei were then encapsulated into droplets together with barcording reagents, i.e. beads linked to barcode oligonucleotides, gap-filling polymerases and PCR reagents. The oligonucleotides were then released from the bead followed by transposase removal from the DNA. The ends of the DNA fragments were then blunt-ended through gap-filling. DNA was then denatured followed by 9 rounds of PCR. At each round of PCR, barcode oligonucleotides tag the nuclear fragments through annealing and polymerase extension reactions. If there are two or more beads per droplet, at each PCR cycle, either of the two barcode oligonucleotides may participate in the tagging reaction. At the end of PCR cycling and after sequencing the fragments, the start and stop sites of the barcoded fragment pool are compared across the barcode space. The co-localization of two barcodes and their respective originating beads were assigned to the same droplet provided a high jaccard index was found using an alternative method as is described in US 2020/0056231 A1. These co-localized beads to single droplets were categorized as "true" as shown in FIG. 9. The sequenced fragments were then compared to each other to identify the number of adjacent reverse complements and at what position relative to each other. As shown in FIG. 9, there was a high proportion of adjacent reverse complements at position 9 in the "true" category, where beads were co-localized to droplets using the orthogonal method mentioned above, thus demonstrating the feasibility of the method. The position 9 was predicted based on the gap produced from the transposition reaction as shown in FIGs. 2 and 5.

It is understood that the examples and embodiments described herein are for illustrative purposes only and that various modifications or changes in light thereof will be suggested to persons skilled in the art.

## Claims

1. A method of deconvoluting sequencing reads from partitions, the method comprising,
performing tagmentation of nucleic acids in permeabilized cells in a mixture, thereby forming at least one cleavage site in a target nucleic acid from one of the cells to form a first nucleic acid fragment and a second nucleic acid fragment, wherein the first and second nucleic acid fragments have at the cleavage site a single-stranded 9 nucleotide sequence, which are complementary to each other, linked to a transposase oligonucleotide delivered by a tagmentation transposase, wherein the transposase oligonucleotide has a double-stranded portion, and a single-stranded 5' portion comprising a universal sequence;
forming a plurality of partitions from the mixture and a plurality of beads and the permeabilized cells, wherein one of the partitions comprise the first nucleic acid fragment and second nucleic acid fragment and at least two beads, wherein the beads are linked to 5' ends of a plurality of clonal barcoding oligonucleotides, the barcoding oligonucleotides comprising a 5' PCR handle sequence, a 3' capture sequence and a barcode sequence unique to the bead to which the barcode oligonucleotide is linked, wherein the 3' capture sequence comprises a copy of said universal sequence;
gap-filling the single-stranded 5' portion of the transposase oligonucleotide to form a reverse complement of the 5' portion and gap-filling the 9 nucleotide sequences, wherein the gap filling comprises using a polymerase to insert nucleotides using the single stranded sequences as a template;
hybridizing the 3' capture sequence of different barcoding oligonucleotides from different beads to the reverse complement of the 5' portion on the first and second nucleic acid fragments and extending the 3' capture sequence of the different barcoding oligonucleotides in a template-dependent manner with a polymerase to form barcoded first and second nucleic acid fragments;
optionally combining the partitions into a bulk solution;
amplifying the barcoded first and second nucleic acid fragments with primers that hybridize to the PCR handle sequences;
generating sequencing reads from the amplified barcoded first and second nucleic acid fragments, wherein the sequencing reads include the barcode sequence, the 9 nucleotide sequence and at least a portion of the nucleic acid fragment from the cell;
identifying in the sequence reads the genomic location relative to the nucleic acid fragment and sequence identity of the 9 nucleotide sequence; and
determining sequencing reads having barcodes from the amplified barcoded first and second barcoding oligonucleotides were from the same partition if the 9 nucleotide sequences in the sequencing reads are reverse complementary sequences and the 9 nucleotide sequences in the sequencing reads are 5' to adjacent genomic positions.

2. The method of claim 1, wherein the nucleic acids in the permeabilized cells
(i) are chromosomal DNA and different chromosomal sequences differ in how accessible the different chromosomal sequences are to the transposase; or
(ii) have been stripped of histones.

3. The method of claim 1, wherein the single-stranded 5' portion of the transposase oligonucleotide comprises (ii) a unique molecular identifier barcode sequence, preferably the unique molecular barcode sequence is 4-10 bp long.

4. The method of claim 1 or 3, wherein the single-stranded 5' portion of the transposase oligonucleotide comprises a multiplexing identifier sequence that distinguishes different samples, preferably the multiplexing identifier sequence is 4-10 bp long.

5. The method of claim 1, wherein the nucleic acids in permeabilized cells are DNA, preferably
(i) the method comprises forming first strand cDNAs or double-stranded cDNAs in the permeabilized cells and the nucleic acids comprise cDNA; or
(ii) the DNA is cellular genomic DNA.

6. A method of deconvoluting sequencing reads from partitions, the method comprising,
performing tagmentation of nucleic acids in permeabilized cells in a mixture, thereby forming at least one cleavage site in a target nucleic acid from one of the cells to form a first nucleic acid fragment and a second nucleic acid fragment, wherein the first and second nucleic acid fragments have at the cleavage site a single-stranded 9 nucleotide sequence, which are complementary to each other, linked to a transposase oligonucleotide delivered by a tagmentation transposase, wherein the transposase oligonucleotide has a double-stranded portion, and a single-stranded 5' portion having a 5' phosphorylated end;
forming a plurality of partitions from the mixture, bridging oligonucleotides, and a plurality of beads and the permeabilized cells, wherein one of the partitions comprise the first nucleic acid fragment and second nucleic acid fragment and at least two beads, wherein the beads are linked to 5' ends of a plurality of clonal barcoding oligonucleotides, the barcoding oligonucleotides comprising a 5' PCR handle sequence, a 3' capture sequence and a barcode sequence unique to the 3' to which the barcode oligonucleotide is linked, and wherein the bridging oligonucleotides comprise (i) a 3' end sequence complementary to the 3' capture sequence of the clonal barcoding oligonucleotides and (ii) a 5' end sequence complementary to the universal sequence of the single-stranded 5' portion of the transposase oligonucleotide;
in the partitions, tagging the first nucleic acid fragment with a barcoding oligonucleotide from a first bead and tagging the second nucleic acid fragment with a barcoding oligonucleotide from a second bead, wherein the tagging comprises hybridizing the 3' capture sequences of the clonal barcoding oligonucleotides to the 3' end sequences of the bridging oligonucleotides and hybridizing the 5' end sequences of the bridging oligonucleotides to the single-stranded 5' portion delivered by the transposase to the first nucleic acid fragment and the second nucleic acid fragment, thereby forming barcoded first and second nucleic acids;
optionally combining the partitions into a bulk solution;
ligating the 3' capture sequences of the clonal barcoding oligonucleotides to the single-stranded 5' portion delivered by the transposase to the first nucleic acid fragment and the second nucleic acid fragment;
gap-filling the 9 nucleotide sequence and the single stranded portion of the bead oligo ligated to transposase adapter, wherein the gap filling comprises using a polymerase to insert nucleotides using the single stranded sequences as a template; amplifying the barcoded first and second nucleic acid fragments with primers that hybridize to the PCR handle sequences;
generating sequencing reads from the amplified barcoded first and second nucleic acid fragments, wherein the sequencing reads include the barcode sequence, the 9 nucleotide duplication sequence and at least a portion of the nucleic acid fragment from the cell;
identifying in the sequence reads the genomic location relative to the nucleic acid fragment and sequence identity of the 9 nucleotide sequence; and
determining sequencing reads having barcodes from the amplified barcoded first and second barcoding oligonucleotides were from the same partition if the 9 nucleotide sequences in the sequencing reads are reverse complementary sequences and the 9 nucleotide sequences in the sequencing reads are 5' to adjacent genomic positions.

7. The method of claim 6, wherein
(i) the nucleic acids in the permeabilized cells are chromosomal DNA and different chromosomal sequences differ in how accessible the different chromosomal sequences are to the transposase; or
(ii) the nucleic acids in the permeabilized cells have been stripped of histones; or
(iii) the partitions further contain a proteinase, surfactant or chaotropic agent; or
(iv) the single-stranded 5' portion of the transposase oligonucleotide comprises (a) a sequence complementary to the 5' end sequences of the bridging oligonucleotides and (b) a unique molecular identifier barcode sequence, preferably the unique molecular barcode sequences is 4-10 bp long.

8. The method of claim 6, wherein
(i) the ligating occurs in the partitions, preferably the partitions are combined after the ligating; or
(ii) the method comprises combining the partitions into a bulk solution, preferably the ligating occurs in the bulk solution.

9. The method of claim 6, wherein the nucleic acids in permeabilized cells are DNA, preferably
(i) the method comprises forming first strand cDNAs or double-stranded cDNAs in the permeabilized cells and the nucleic acids comprise cDNA; or
(ii) the DNA is cellular genomic DNA.

10. The method of any one of claims 1-3 and 8-9, wherein
(i) the partitions are droplets in a water-in-oil emulsion; or
(ii) the partitions are microwells.

11. The method of any one of claims 1-10, wherein the tagging further comprises tagging nucleic acids in the cells such that two or more types of nucleic acids are tagged and subsequently sequenced, preferably the two types of nucleic acids are genomic DNA or cDNA.

12. A method of determining relative position of beads on a solid support, the method comprising
providing a tissue section fixed to a solid support;
performing tagmentation of nucleic acids in the tissue section, thereby forming at least one cleavage site in a target nucleic acid within the tissue section to form a first nucleic acid fragment and a second nucleic acid fragment, wherein the first and second nucleic acid fragments receive at the cleavage site a single-stranded 9 nucleotide duplication sequence linked to a transposase oligonucleotide with a double-stranded portion and a single-stranded 5' portion delivered by the transposase;
contacting to the tagmented nucleic acid in the tissue section bridging oligonucleotides and oligonucleotides from a plurality of beads, wherein the beads are linked to 5' ends of a plurality of clonal barcoding oligonucleotides, the barcoding oligonucleotides comprising a 5' PCR handle sequence, a 3' capture sequence and a barcode sequence unique to the bead to which the barcode oligonucleotide is linked, wherein the oligonucleotides are released from the beads and wherein the bridging oligonucleotides comprise (i) a 3' end sequence complementary to the 3' capture sequence of the clonal barcoding oligonucleotides and (ii) a 5' end sequence complementary to the single-stranded 5' portion of the transposase oligonucleotide; tagging the first nucleic acid fragment with a barcoding oligonucleotide from a first bead and tagging the second nucleic acid fragment with a barcoding oligonucleotide from a second bead, wherein the tagging comprises hybridizing the 3' capture sequences of the clonal barcoding oligonucleotides to the 3' end sequences of the bridging oligonucleotides and hybridizing the 5' end sequences of the bridging oligonucleotides to the single-stranded 5' portion delivered by the transposase to the first nucleic acid fragment and the second nucleic acid fragment, thereby forming barcoded first and second nucleic acids;
optionally washing the barcoded first and second nucleic acids from the planar solid support;
ligating the 3' capture sequences of the clonal barcoding oligonucleotides to the single-stranded 5' portion delivered by the transposase to the first nucleic acid fragment and the second nucleic acid fragment;
optionally washing the barcoded first and second nucleic acids from the planar solid support if the barcoded first and second nucleic acids have not been previously washed from the planar support;
gap-filling the 9 nucleotide sequence, wherein the gap filling comprises using a polymerase to insert nucleotides using the single stranded sequences as a template; amplifying the barcoded first and second nucleic acid fragments with primers that hybridize to the PCR handle sequences;
generating sequencing reads from the amplified barcoded first and second nucleic acid fragments, wherein the sequencing reads include the barcode sequence, the 9 nucleotide sequence and at least a portion of the nucleic acid fragment from the cell;
identifying in the sequence reads the genomic location relative to the nucleic acid fragment and sequence identity of the 9 nucleotide duplication sequence; and
determining sequencing reads having barcodes from the amplified barcoded first and second barcoding oligonucleotides were from adjacent beads on the tissue section if the 9 nucleotide sequences in the sequencing reads are reverse complementary sequences and the 9 nucleotide sequences in the sequencing reads are 5' to adjacent genomic positions.

13. The method of claim 12, wherein
(i) washing of the barcoded first and second nucleic acids from the planar solid support occurs before the ligating and the ligating occurs in a solution washed from the planar solid support; or
(ii) the ligating occurs in a solution on the planar solid support and washing of the barcoded first and second nucleic acids from the planar solid support occurs after the ligating and before the gap filling.

14. The method of claim 12 or 13, wherein the method is repeated for a plurality (e.g., at least 3, 5 ,10, 20, 50, 100 or more) beads linked to 5' ends of a plurality of clonal barcoding oligonucleotides, the barcoding oligonucleotides comprising a 5' PCR handle sequence, a 3' capture sequence and a barcode sequence unique to the bead to which the barcode oligonucleotide is linked, and wherein the bridging oligonucleotides comprise (i) a 3' end sequence complementary to the 3' capture sequence of the clonal barcoding oligonucleotides and (ii) a 5' end sequence complementary to the single-stranded 5' portion of the transposase oligonucleotide, thereby determining sequencing reads having barcodes from amplified barcoded barcoding oligonucleotides were from adjacent beads for at least a portion (e.g., at least 5%, 10%, 20%, 40%, 50% or more) of the beads on the tissue section, thereby generating a 2-D map of beads on the tissue section.

15. The method of any one of claims 12-14, wherein the tagging further comprises tagging nucleic acids in the tissue section such that two or more types of nucleic acids are tagged and subsequently sequenced, preferably the two types of nucleic acids are genomic DNA or cDNA.

## Patentansprüche

1. Verfahren zur Dekonvolution von Sequenzierungsdaten aus Partitionen, wobei das Verfahren umfasst:
das Durchführen einer Tagmentierung von Nucleinsäuren in permeabilisierten Zellen in einem Gemisch, wodurch wenigstens eine Spaltungsstelle in einer Zielnucleinsäure aus einer der Zellen gebildet wird, so dass ein erstes Nucleinsäurefragment und ein zweites Nucleinsäurefragment entstehen, wobei das erste und das zweite Nucleinsäurefragment an der Spaltungsstelle jeweils eine aus 9 Nucleotiden bestehende einzelsträngige Sequenz aufweisen, die komplementär zueinander sind und an ein Transposase-Oligonucleotid gebunden sind, das von einer Tagmentierungs-Transposase bereitgestellt wird, wobei das Transposase-Oligonucleotid einen doppelsträngigen Teil und einen einzelsträngigen 5'-Teil, der eine universelle Sequenz umfasst, aufweist;
das Bilden einer Vielzahl von Partitionen aus dem Gemisch und einer Vielzahl von Beads und den permeabilisierten Zellen, wobei eine der Partitionen das erste Nucleinsäurefragment und das zweite Nucleinsäurefragment und wenigstens zwei Beads umfasst, wobei die Beads an 5'-Enden einer Vielzahl von klonalen Barcoding-Oligonucleotiden gebunden sind, wobei die Barcoding-Oligonucleotide eine 5'-PCR-Handle-Sequenz, eine 3'-Capture-Sequenz und eine für das Bead, an das das Barcoding-Oligonucleotid gebunden ist, einzigartige Barcode-Sequenz umfassen, wobei die 3'-Capture-Sequenz eine Kopie der universellen Sequenz umfasst;
das Auffüllen der Lücke im einzelsträngigen 5'-Teil des Transposase-Oligonucleotids unter Bildung eines reversen Komplements des 5'-Teils und das Auffüllen der Lücken in den aus 9 Nucleotiden bestehenden Sequenzen, wobei das Auffüllen der Lücken die Verwendung einer Polymerase umfasst, um Nucleotide zu inserieren, wobei die einzelsträngigen Sequenzen als Matrize dienen;
das Hybridisieren der 3'-Capture-Sequenz von verschiedenen Barcoding-Oligonucleotiden aus verschiedenen Beads mit dem reversen Komplement des 5'-Teils an dem ersten und zweiten Nucleinsäurefragment und das matrizenabhängige Verlängern der 3'-Capture-Sequenz der verschiedenen Barcoding-Oligonucleotide mit einer Polymerase unter Bildung eines ersten und eines zweiten Barcode-Nucleinsäurefragments;
gegebenenfalls das Kombinieren der Partitionen zu einer Sammellösung;
das Amplifizieren des ersten und zweiten Barcode-Nucleinsäurefragments mit Primern, die an die PCR-Handle-Sequenzen hybridisieren;
das Erzeugen von Sequenzierungsdaten aus den amplifizierten ersten und zweiten Barcode-Nucleinsäurefragmenten, wobei die Sequenzierungsdaten die Barcode-Sequenz, die aus 9 Nucleotiden bestehende Sequenz und wenigstens einen Teil des Nucleinsäurefragments aus der Zelle umfassen;
das Identifizieren des genomischen Ortes relativ zu dem Nucleinsäurefragment und der Sequenzidentität der aus 9 Nucleotiden bestehenden Sequenz in den Sequenzierungsdaten; und
das Bestimmen, dass Sequenzierungsdaten mit Barcodes von den amplifizierten, mit einem Barcode versehenen ersten und zweiten Barcoding-Oligonucleotiden aus derselben Partition stammen, wenn die aus 9 Nucleotiden bestehenden Sequenzen in den Sequenzierungsdaten revers-komplementäre Sequenzen sind und die aus 9 Nucleotiden bestehenden Sequenzen in den Sequenzierungsdaten auf der 5'-Seite von benachbarten genomischen Positionen liegen.

2. Verfahren gemäß Anspruch 1, wobei die Nucleinsäuren in den permeabilisierten Zellen
(i) chromosomale DNA sind und sich verschiedene chromosomale Sequenzen darin unterscheiden, wie gut zugänglich die verschiedenen chromosomalen Sequenzen für die Transposase sind; oder
(ii) von Histonen befreit sind.

3. Verfahren gemäß Anspruch 1, wobei der einzelsträngige 5'-Teil des Transposase-Oligonucleotids (ii) eine einzigartige, als molekularer Identifikator geeignete Barcode-Sequenz umfasst, vorzugsweise die einzigartige molekulare Barcode-Sequenz 4-10 bp lang ist.

4. Verfahren gemäß Anspruch 1 oder 3, wobei der einzelsträngige 5'-Teil des Transposase-Oligonucleotids eine Multiplexing-Identifikationssequenz umfasst, mit der sich verschiedene Proben unterscheiden lassen, vorzugsweise die Multiplexing-Identifikationssequenz 4-10 bp lang ist.

5. Verfahren gemäß Anspruch 1, wobei die Nucleinsäuren in permeabilisierten Zellen DNA sind, vorzugsweise
(i) das Verfahren das Bilden von cDNAs des ersten Strangs oder von doppelsträngigen cDNAs in den permeabilisierten Zellen umfasst und die Nucleinsäuren cDNA umfassen; oder
(ii) die DNA zelluläre genomische DNA ist.

6. Verfahren zur Dekonvolution von Sequenzierungsdaten aus Partitionen, wobei das Verfahren umfasst:
das Durchführen einer Tagmentierung von Nucleinsäuren in permeabilisierten Zellen in einem Gemisch, wodurch wenigstens eine Spaltungsstelle in einer Zielnucleinsäure aus einer der Zellen gebildet wird, so dass ein erstes Nucleinsäurefragment und ein zweites Nucleinsäurefragment entstehen, wobei das erste und das zweite Nucleinsäurefragment an der Spaltungsstelle jeweils eine aus 9 Nucleotiden bestehende einzelsträngige Sequenz aufweisen, die komplementär zueinander sind und an ein Transposase-Oligonucleotid gebunden sind, das von einer Tagmentierungs-Transposase bereitgestellt wird, wobei das Transposase-Oligonucleotid einen doppelsträngigen Teil und einen einzelsträngigen 5'-Teil, der ein 5'-phosphoryliertes Ende hat, aufweist;
das Bilden einer Vielzahl von Partitionen aus dem Gemisch, verbrückenden Oligonucleotiden und einer Vielzahl von Beads und den permeabilisierten Zellen, wobei eine der Partitionen das erste Nucleinsäurefragment und das zweite Nucleinsäurefragment und wenigstens zwei Beads umfasst, wobei die Beads an 5'-Enden einer Vielzahl von klonalen Barcoding-Oligonucleotiden gebunden sind, wobei die Barcoding-Oligonucleotide eine 5'-PCR-Handle-Sequenz, eine 3'-Capture-Sequenz und eine für das 3'-Ende, an das das Barcoding-Oligonucleotid gebunden ist, einzigartige Barcode-Sequenz umfassen und wobei die verbrückenden Oligonucleotide (i) eine 3'-Ende-Sequenz, die zu der 3'-Capture-Sequenz der klonalen Barcoding-Oligonucleotide komplementär ist, und (ii) eine 5'-Ende-Sequenz, die zu der universellen Sequenz des einzelsträngigen 5'-Teils des Transposase-Oligonucleotids komplementär ist, umfassen;
in den Partitionen: das Markieren des ersten Nucleinsäurefragments mit einem Barcoding-Oligonucleotid aus einem ersten Bead und das Markieren des zweiten Nucleinsäurefragments mit einem Barcoding-Oligonucleotid aus einem zweiten Bead, wobei das Markieren das Hybridisieren der 3'-Capture-Sequenzen der klonalen Barcoding-Oligonucleotide mit den 3'-Ende-Sequenzen der verbrückenden Oligonucleotide und das Hybridisieren der 5'-Ende-Sequenzen der verbrückenden Oligonucleotide mit dem einzelsträngigen 5'-Teil, der durch die Transposase für das erste Nucleinsäurefragment und das zweite Nucleinsäurefragment bereitgestellt wurde, umfasst, wodurch eine erste und eine zweite Barcode-Nucleinsäure entsteht;
gegebenenfalls das Kombinieren der Partitionen zu einer Sammellösung;
das Ligieren der 3'-Capture-Sequenzen der klonalen Barcoding-Oligonucleotide mit dem einzelsträngigen 5'-Teil, der durch die Transposase für das erste Nucleinsäurefragment und das zweite Nucleinsäurefragment bereitgestellt wurde;
das Auffüllen der Lücke in der aus 9 Nucleotiden bestehenden Sequenz und in dem einzelsträngigen Teil des an den Transposase-Adapter ligierten Bead-Oligo, wobei das Auffüllen der Lücken die Verwendung einer Polymerase umfasst, um Nucleotide zu inserieren, wobei die einzelsträngigen Sequenzen als Matrize dienen;
das Amplifizieren des ersten und zweiten Barcode-Nucleinsäurefragments mit Primern, die an die PCR-Handle-Sequenzen hybridisieren;
das Erzeugen von Sequenzierungsdaten aus den amplifizierten ersten und zweiten Barcode-Nucleinsäurefragmenten, wobei die Sequenzierungsdaten die Barcode-Sequenz, die aus 9 Nucleotiden bestehende Duplikatsequenz und wenigstens einen Teil des Nucleinsäurefragments aus der Zelle umfassen;
das Identifizieren des genomischen Ortes relativ zu dem Nucleinsäurefragment und der Sequenzidentität der aus 9 Nucleotiden bestehenden Sequenz in den Sequenzierungsdaten; und
das Bestimmen, dass Sequenzierungsdaten mit Barcodes von den amplifizierten, mit einem Barcode versehenen ersten und zweiten Barcoding-Oligonucleotiden aus derselben Partition stammen, wenn die aus 9 Nucleotiden bestehenden Sequenzen in den Sequenzierungsdaten revers-komplementäre Sequenzen sind und die aus 9 Nucleotiden bestehenden Sequenzen in den Sequenzierungsdaten auf der 5'-Seite von benachbarten genomischen Positionen liegen.

7. Verfahren gemäß Anspruch 6, wobei
(i) die Nucleinsäuren in den permeabilisierten Zellen chromosomale DNA sind und sich verschiedene chromosomale Sequenzen darin unterscheiden, wie gut zugänglich die verschiedenen chromosomalen Sequenzen für die Transposase sind; oder
(ii) die Nucleinsäuren in den permeabilisierten Zellen von Histonen befreit sind; oder
(iii) die Partitionen ferner eine Proteinase, ein Tensid oder ein chaotropes Mittel enthalten; oder
(iv) der einzelsträngige 5'-Teil des Transposase-Oligonucleotids (a) eine zu den 5'-Ende-Sequenzen der verbrückenden Oligonucleotide komplementäre Sequenz und (b) eine einzigartige, als molekularer Identifikator geeignete Barcode-Sequenz umfasst, vorzugsweise die einzigartige molekulare Barcode-Sequenz 4-10 bp lang ist.

8. Verfahren gemäß Anspruch 6, wobei
(i) das Ligieren in den Partitionen erfolgt, vorzugsweise die Partitionen nach dem Ligieren vereinigt werden; oder
(ii) das Verfahren das Kombinieren der Partitionen zu einer Sammellösung umfasst, vorzugsweise das Ligieren in der Sammellösung erfolgt.

9. Verfahren gemäß Anspruch 6, wobei die Nucleinsäuren in permeabilisierten Zellen DNA sind, vorzugsweise
(i) das Verfahren das Bilden von cDNAs des ersten Strangs oder von doppelsträngigen cDNAs in den permeabilisierten Zellen umfasst und die Nucleinsäuren cDNA umfassen; oder
(ii) die DNA zelluläre genomische DNA ist.

10. Verfahren gemäß einem der Ansprüche 1-3 und 8-9, wobei
(i) die Partitionen Tröpfchen in einer Wasser-in-Öl-Emulsion sind; oder
(ii) die Partitionen Mikrowells sind.

11. Verfahren gemäß einem der Ansprüche 1-10, wobei das Markieren ferner das Markieren von Nucleinsäuren in den Zellen in einer solchen Weise umfasst, dass zwei oder mehr Arten von Nucleinsäuren markiert und anschließend sequenziert werden, es sich vorzugsweise bei den zwei Arten von Nucleinsäuren um genomische DNA oder cDNA handelt.

12. Verfahren zum Bestimmen der relativen Position von Beads auf einem festen Träger, wobei das Verfahren umfasst:
das Bereitstellen eines Gewebeschnitts, der an einem festen Träger fixiert ist;
das Durchführen einer Tagmentierung von Nucleinsäuren in dem Gewebeschnitt, wodurch wenigstens eine Spaltungsstelle in einer Zielnucleinsäure innerhalb des Gewebeschnitts gebildet wird, so dass ein erstes Nucleinsäurefragment und ein zweites Nucleinsäurefragment entstehen, wobei das erste und das zweite Nucleinsäurefragment an der Spaltungsstelle eine einzelsträngige, aus 9 Nucleotiden bestehende Duplikatsequenz, die an ein Transposase-Oligonucleotid gebunden ist, mit einem doppelsträngigen Teil und einem von der Transposase bereitgestellten einzelsträngigen 5'-Teil erhalten;
das In-Kontakt-Bringen von verbrückenden Oligonucleotiden und von Oligonucleotiden aus einer Vielzahl von Beads mit der tagmentierten Nucleinsäure in dem Gewebeschnitt, wobei die Beads an 5'-Enden einer Vielzahl von klonalen Barcoding-Oligonucleotiden gebunden sind, wobei die Barcoding-Oligonucleotide eine 5'-PCR-Handle-Sequenz, eine 3'-Capture-Sequenz und eine für das Bead, an das das Barcoding-Oligonucleotid gebunden ist, einzigartige Barcode-Sequenz umfassen, wobei die Oligonucleotide von den Beads freigesetzt werden und wobei die verbrückenden Oligonucleotide (i) eine 3'-Ende-Sequenz, die zu der 3'-Capture-Sequenz der klonalen Barcoding-Oligonucleotide komplementär ist, und (ii) eine 5'-Ende-Sequenz, die zu dem einzelsträngigen 5'-Teil des Transposase-Oligonucleotids komplementär ist, umfassen;
das Markieren des ersten Nucleinsäurefragments mit einem Barcoding-Oligonucleotid aus einem ersten Bead und das Markieren des zweiten Nucleinsäurefragments mit einem Barcoding-Oligonucleotid aus einem zweiten Bead, wobei das Markieren das Hybridisieren der 3'-Capture-Sequenzen der klonalen Barcoding-Oligonucleotide mit den 3'-Ende-Sequenzen der verbrückenden Oligonucleotide und das Hybridisieren der 5'-Ende-Sequenzen der verbrückenden Oligonucleotide mit dem einzelsträngigen 5'-Teil, der durch die Transposase für das erste Nucleinsäurefragment und das zweite Nucleinsäurefragment bereitgestellt wurde, umfasst, wodurch eine erste und eine zweite Barcode-Nucleinsäure entsteht;
gegebenenfalls das Auswaschen der ersten und zweiten Barcode-Nucleinsäuren aus dem planaren festen Träger;
das Ligieren der 3'-Capture-Sequenzen der klonalen Barcoding-Oligonucleotide mit dem einzelsträngigen 5'-Teil, der durch die Transposase für das erste Nucleinsäurefragment und das zweite Nucleinsäurefragment bereitgestellt wurde;
gegebenenfalls das Auswaschen der ersten und zweiten Barcode-Nucleinsäuren aus dem planaren festen Träger, wenn die erste und zweite Barcode-Nucleinsäure bisher noch nicht aus dem planaren Träger ausgewaschen wurden;
das Auffüllen der Lücke in der aus 9 Nucleotiden bestehenden Sequenz, wobei das Auffüllen der Lücke die Verwendung einer Polymerase umfasst, um Nucleotide zu inserieren, wobei die einzelsträngigen Sequenzen als Matrize dienen;
das Amplifizieren des ersten und zweiten Barcode-Nucleinsäurefragments mit Primern, die an die PCR-Handle-Sequenzen hybridisieren;
das Erzeugen von Sequenzierungsdaten aus den amplifizierten ersten und zweiten Barcode-Nucleinsäurefragmenten, wobei die Sequenzierungsdaten die Barcode-Sequenz, die aus 9 Nucleotiden bestehende Sequenz und wenigstens einen Teil des Nucleinsäurefragments aus der Zelle umfassen;
das Identifizieren des genomischen Ortes relativ zu dem Nucleinsäurefragment und der Sequenzidentität der aus 9 Nucleotiden bestehenden Duplikatsequenz in den Sequenzierungsdaten; und
das Bestimmen, dass Sequenzierungsdaten mit Barcodes von den amplifizierten, mit einem Barcode versehenen ersten und zweiten Barcoding-Oligonucleotiden aus benachbarten Beads auf dem Gewebeschnitt stammen, wenn die aus 9 Nucleotiden bestehenden Sequenzen in den Sequenzierungsdaten revers-komplementäre Sequenzen sind und die aus 9 Nucleotiden bestehenden Sequenzen in den Sequenzierungsdaten auf der 5'-Seite von benachbarten genomischen Positionen liegen.

13. Verfahren gemäß Anspruch 12, wobei
(i) das Auswaschen der ersten und zweiten Barcode-Nucleinsäuren aus dem planaren festen Träger vor dem Ligieren erfolgt und das Ligieren in einer aus dem planaren festen Träger ausgewaschenen Lösung erfolgt; oder
(ii) das Ligieren in einer Lösung auf dem planaren festen Träger erfolgt und das Auswaschen der ersten und zweiten Barcode-Nucleinsäuren aus dem planaren festen Träger nach dem Ligieren und vor dem Auffüllen der Lücke erfolgt.

14. Verfahren gemäß Anspruch 12 oder 13, wobei das Verfahren mit einer Vielzahl von (z.B. wenigstens 3, 5, 10, 20, 50, 100 oder mehr) Beads wiederholt wird, die an 5'-Enden einer Vielzahl von klonalen Barcoding-Oligonucleotiden gebunden sind, wobei die Barcoding-Oligonucleotide eine 5'-PCR-Handle-Sequenz, eine 3'-Capture-Sequenz und eine für das Bead, an das das Barcoding-Oligonucleotid gebunden ist, einzigartige Barcode-Sequenz umfassen und wobei die verbrückenden Oligonucleotide (i) eine 3'-Ende-Sequenz, die zu der 3'-Capture-Sequenz der klonalen Barcoding-Oligonucleotide komplementär ist, und (ii) eine 5'-Ende-Sequenz, die zu dem einzelsträngigen 5'-Teil des Transposase-Oligonucleotids komplementär ist, umfassen, wodurch bestimmt wird, dass Sequenzierungsdaten mit Barcodes von amplifizierten, mit einem Barcode versehenen Barcoding-Oligonucleotiden zu wenigstens einem Teil (z.B. wenigstens 5%, 10%, 20%, 40%, 50% oder mehr) der Beads auf dem Gewebeschnitt aus benachbarten Beads stammen, wodurch eine 2D-Karte von Beads auf dem Gewebeschnitt erzeugt wird.

15. Verfahren gemäß einem der Ansprüche 12-14, wobei das Markieren ferner das Markieren von Nucleinsäuren in dem Gewebeschnitt in einer solchen Weise umfasst, dass zwei oder mehr Arten von Nucleinsäuren markiert und anschließend sequenziert werden, es sich vorzugsweise bei den zwei Arten von Nucleinsäuren um genomische DNA oder cDNA handelt.

## Revendications

1. Procédé de déconvolution de lectures de séquençage à partir de partitions, le procédé comprenant,
la réalisation de la tagmentation d'acides nucléiques dans des cellules perméabilisées dans un mélange, formant ainsi au moins un site de clivage dans un acide nucléique cible à partir de l'une des cellules pour former un premier fragment d'acide nucléique et un second fragment d'acide nucléique, dans lequel les premier et second fragments d'acide nucléique présentent au site de clivage une séquence de 9 nucléotides à un seul brin, qui sont complémentaires les uns aux autres, liés à un oligonucléotide transposase délivré par une transposase de tagmentation, dans lequel l'oligonucléotide transposase présente une portion à double brin, et une portion 5' à un seul brin comprenant une séquence universelle ;
la formation d'une pluralité de partitions à partir du mélange et d'une pluralité de billes et des cellules perméabilisées, dans lequel l'une des partitions comprend le premier fragment d'acide nucléique et le second fragment d'acide nucléique et au moins deux billes, dans lequel les billes sont liées à des extrémités 5' d'une pluralité d'oligonucléotides de codage à barres clonaux, les oligonucléotides de codage à barres comprenant une séquence de poignée PCR 5', une séquence de capture 3' et une séquence de code-barres unique à la bille à laquelle l'oligonucléotide à code-barres est lié, dans lequel la séquence de capture 3' comprend une copie de ladite séquence universelle ;
le comblement de brèche(s) de la portion 5' à un seul brin de l'oligonucléotide transposase pour former un complémentaire inverse de la portion 5' et le comblement de brèche(s) des séquences de 9 nucléotides, dans lequel le comblement de brèche(s) comprend l'utilisation d'une polymérase pour insérer des nucléotides en utilisant les séquences à un seul brin comme matrice ;
l'hybridation de la séquence de capture 3' de différents oligonucléotides de codage à barres de billes différentes au complément inverse de la portion 5' sur les premier et second fragments d'acide nucléique et l'extension de la séquence de capture 3' des différents oligonucléotides de codage à barres de manière dépendante de la matrice avec une polymérase pour former des premier et second fragments d'acide nucléique à code-barres ;
facultativement la combinaison des partitions en une solution en vrac ;
l'amplification des premier et second fragments d'acide nucléique à code-barres avec des amorces qui s'hybrident aux séquences de poignée PCR ;
la génération de lectures de séquençage à partir des premier et second fragments d'acide nucléique à code-barres amplifiés, dans lequel les lectures de séquençage incluent la séquence de codes-barres, la séquence de 9 nucléotides et au moins une portion du fragment d'acide nucléique à partir de la cellule ;
l'identification, dans les lectures de séquençage, de l'emplacement génomique par rapport au fragment d'acide nucléique et l'identité de séquence de la séquence de 9 nucléotides ; et
la détermination que des lectures de séquençage ayant des codes-barres à partir des premier et second oligonucléotides de codage à barres amplifiés provenaient de la même partition si les séquences de 9 nucléotides dans les lectures de séquençage sont des séquences complémentaires inverses et les séquences de 9 nucléotides dans les lectures de séquençage sont situées en 5' à des positions génomiques adjacentes.

2. Procédé selon la revendication 1, dans lequel les acides nucléiques dans les cellules perméabilisées
(i) sont de l'ADN chromosomique et différentes séquences chromosomiques diffèrent dans la mesure où les différentes séquences chromosomiques sont accessibles à la transposase ; ou
(ii) ont été dépourvus d'histones.

3. Procédé selon la revendication 1, dans lequel la portion 5' à un seul brin de l'oligonucléotide transposase comprend (ii) une séquence de codes-barres d'identifiant moléculaire unique, de préférence la séquence de codes-barres moléculaire unique est de 4 à 10 pb de long.

4. Procédé selon la revendication 1 ou 3, dans lequel la portion 5' à un seul brin de l'oligonucléotide transposase comprend une séquence d'identifiant de multiplexage qui distingue différents échantillons, de préférence la séquence d'identifiant de multiplexage est de 4 à 10 pb de long.

5. Procédé selon la revendication 1, dans lequel les acides nucléiques dans des cellules perméabilisées sont de l'ADN, de préférence
(i) le procédé comprend la formation d'ADNc à premier brin ou d'ADNc à double brin dans les cellules perméabilisées et les acides nucléiques comprennent de l'ADNc ; ou
(ii) l'ADN est un ADN génomique cellulaire.

6. Procédé de déconvolution de lectures de séquençage à partir de partitions, le procédé comprenant,
la réalisation de la tagmentation d'acides nucléiques dans des cellules perméabilisées dans un mélange, formant ainsi au moins un site de clivage dans un acide nucléique cible à partir de l'une des cellules pour former un premier fragment d'acide nucléique et un second fragment d'acide nucléique, dans lequel les premier et second fragments d'acide nucléique présentent au site de clivage une séquence de 9 nucléotides à un seul brin, qui sont complémentaires les uns aux autres, liés à un oligonucléotide transposase délivré par une transposase de tagmentation, dans lequel l'oligonucléotide transposase a une portion à double brin, et une portion 5' à un seul brin ayant une extrémité phosphorylée 5' ;
la formation d'une pluralité de partitions à partir du mélange, d'oligonucléotides de pontage, et d'une pluralité de billes et des cellules perméabilisées, dans lequel l'une des partitions comprend le premier fragment d'acide nucléique et le second fragment d'acide nucléique et au moins deux billes, dans lequel les billes sont liées à des extrémités 5' d'une pluralité d'oligonucléotides de codage à barres clonaux, les oligonucléotides de codage à barres comprenant une séquence de poignée PCR 5', une séquence de capture 3' et une séquence de codes-barres unique aux 3' à laquelle l'oligonucléotide à code-barres est lié, et dans lequel les oligonucléotides de pontage comprennent (i) une séquence terminale 3' complémentaire à la séquence de capture 3' des oligonucléotides de codage à barres clonaux et (ii) une séquence terminale 5' complémentaire à la séquence universelle de la portion 5' à un seul brin de l'oligonucléotide transposase ;
dans les partitions, le marquage du premier fragment d'acide nucléique avec un oligonucléotide de codage à barres à partir d'une première bille et le marquage du second fragment d'acide nucléique avec un oligonucléotide de codage à barres à partir d'une seconde bille, dans lequel le marquage comprend l'hybridation des séquences de capture 3' des oligonucléotides de codage à barres clonaux aux séquences terminales 3' des oligonucléotides de pontage et l'hybridation des séquences terminales 5' des oligonucléotides de pontage à la portion 5' à un seul brin délivrée par la transposase au premier fragment d'acide nucléique et au second fragment d'acide nucléique, formant ainsi des premier et second acides nucléiques à code-barres ;
facultativement la combinaison des partitions en une solution en vrac ;
la ligation des séquences de capture 3' des oligonucléotides de codage à barres clonaux à la portion 5' à un seul brin délivrée par la transposase au premier fragment d'acide nucléique et au second fragment d'acide nucléique ;
le comblement de brèche(s) de la séquence de 9 nucléotides et de la portion à un seul brin de l'adaptateur d'oligo-bille ligaturé à la transposase, dans lequel le comblement de brèche(s) comprend l'utilisation d'une polymérase pour insérer des nucléotides en utilisant les séquences à un seul brin comme matrice ; l'amplification des premier et second fragments d'acide nucléique à code-barres avec des amorces qui s'hybrident aux séquences de poignée PCR ;
la génération de lectures de séquençage à partir des premier et second fragments d'acide nucléique à codes-barres amplifiés, dans lequel les lectures de séquençage incluent la séquence de codes-barres, la séquence de duplication de 9 nucléotides et au moins une portion du fragment d'acide nucléique à partir de la cellule ;
l'identification, dans les lectures de séquençage, de l'emplacement génomique par rapport au fragment d'acide nucléique et l'identité de séquence de la séquence de 9 nucléotides ; et
la détermination que des lectures de séquençage ayant des codes-barres à partir des premier et second oligonucléotides de codage à barres amplifiés provenaient de la même partition si les séquences de 9 nucléotides dans les lectures de séquençage sont des séquences complémentaires inverses et les séquences de 9 nucléotides dans les lectures de séquençage sont situées en 5' à des positions génomiques adjacentes.

7. Procédé selon la revendication 6, dans lequel
(i) les acides nucléiques dans les cellules perméabilisées sont de l'ADN chromosomique et différentes séquences chromosomiques diffèrent dans la mesure où les différentes séquences chromosomiques sont accessibles à la transposase ; ou
(ii) les acides nucléiques dans les cellules perméabilisées ont été dépourvus d'histones ; ou
(iii) les partitions contiennent en outre une protéinase, un tensioactif ou un agent chaotropique ; ou
(iv) la portion 5' à un seul brin de l'oligonucléotide transposase comprend (a) une séquence complémentaire aux séquences terminales 5' des oligonucléotides de pontage et (b) une séquence de codes-barres d'identifiant moléculaire unique, de préférence les séquences de codes-barres moléculaires uniques sont d'une longueur de 4 à 10 pb.

8. Procédé selon la revendication 6, dans lequel
(i) la ligation a lieu dans les partitions, de préférence les partitions sont combinées après la ligation ; ou
(ii) le procédé comprend la combinaison des partitions en une solution en vrac, de préférence la ligation se produit dans la solution en vrac.

9. Procédé selon la revendication 6, dans lequel les acides nucléiques dans les cellules perméabilisées sont de l'ADN, de préférence
(i) le procédé comprend la formation d'ADNc à premier brin ou d'ADNc à double brin dans les cellules perméabilisées et les acides nucléiques comprennent de l'ADNc ; ou
(ii) l'ADN est un ADN génomique cellulaire.

10. Procédé selon l'une quelconque des revendications 1 à 3 et 8 et 9, dans lequel
(i) les partitions sont des gouttelettes dans une émulsion eau dans l'huile ; ou
(ii) les partitions sont des micropuits.

11. Procédé selon l'une quelconque des revendications 1 à 10, dans lequel le marquage comprend en outre le marquage d'acides nucléiques dans les cellules de sorte que deux types ou plus d'acides nucléiques sont étiquetés et ensuite séquencés, de préférence les deux types d'acides nucléiques sont l'ADN génomique ou l'ADNc.

12. Procédé de détermination de position relative de billes sur un support solide, le procédé comprenant
la fourniture d'une section tissulaire fixée à un support solide ;
la réalisation d'une tagmentation d'acides nucléiques dans la section tissulaire, formant ainsi au moins un site de clivage dans un acide nucléique cible au sein de la section tissulaire pour former un premier fragment d'acide nucléique et un second fragment d'acide nucléique, dans lequel les premier et second fragments d'acide nucléique reçoivent au site de clivage une séquence de duplication de 9 nucléotides à un seul brin liée à un oligonucléotide transposase avec une portion à double brin et une portion 5' à un seul brin délivrée par la transposase ;
la mise en contact avec l'acide nucléique tagmenté dans la section tissulaire, d'oligonucléotides de pontage et des oligonucléotides à partir d'une pluralité de billes, dans lequel les billes sont liées aux extrémités 5' d'une pluralité d'oligonucléotides de codage à barres clonaux, les oligonucléotides de codage à barres comprenant une séquence de poignée PCR 5', une séquence de capture 3' et une séquence de code-barres unique à la bille à laquelle l'oligonucléotide à code-barres est lié, dans lequel les oligonucléotides sont libérés des billes et dans lequel les oligonucléotides de pontage comprennent (i) une séquence terminale 3' complémentaire à la séquence de capture 3' des oligonucléotides de codage à barres clonaux et (ii) une séquence terminale 5' complémentaire à la portion 5' à un seul brin de l'oligonucléotide transposase ; le marquage du premier fragment d'acide nucléique avec un oligonucléotide de codage à barres à partir d'une première bille et le marquage du second fragment d'acide nucléique avec un oligonucléotide de codage à barres à partir d'une seconde bille, dans lequel le marquage comprend l'hybridation des séquences de capture 3' des oligonucléotides de codage à barres clonaux aux séquences terminales 3' des oligonucléotides de pontage et l'hybridation des séquences terminales 5' des oligonucléotides de pontage à la portion 5' à un seul brin délivrée par la transposase au premier fragment d'acide nucléique et au second fragment d'acide nucléique, formant ainsi des premier et second acides nucléiques à code-barres ;
facultativement le lavage des premier et second acides nucléiques à code-barres à partir du support solide planaire ;
la ligation des séquences de capture 3' des oligonucléotides de codage à barres clonaux à la portion 5' à un seul brin délivrée par la transposase au premier fragment d'acide nucléique et au second fragment d'acide nucléique ;
facultativement le lavage des premier et second acides nucléiques à code-barres à partir du support solide planaire si les premier et second acides nucléiques à code-barres n'ont pas été préalablement lavés à partir du support planaire ;
le comblement de brèche(s) de la séquence de 9 nucléotides, dans lequel le comblement de brèche(s) comprend l'utilisation d'une polymérase pour insérer des nucléotides en utilisant les séquences à un seul brin comme matrice ; l'amplification des premier et second fragments d'acide nucléique à code-barres avec des amorces qui s'hybrident aux séquences de poignée PCR ;
la génération de lectures de séquençage à partir des premier et second fragments d'acide nucléique à codes-barres amplifiés, dans lequel les lectures de séquençage incluent la séquence de codes-barres, la séquence de 9 nucléotides et au moins une portion du fragment d'acide nucléique de la cellule ;
l'identification, dans les lectures de séquençage, de l'emplacement génomique par rapport au fragment d'acide nucléique et l'identité de séquence de la séquence de duplication de 9 nucléotides ; et
la détermination que des lectures de séquençage ayant des codes-barres à partir des premier et second oligonucléotides de codage à barres amplifiés provenaient de billes adjacentes sur la section tissulaire si les séquences de 9 nucléotides dans les lectures de séquençage sont des séquences complémentaires inverses et les séquences de 9 nucléotides dans les lectures de séquençage sont situées en 5' à des positions génomiques adjacentes.

13. Procédé selon la revendication 12, dans lequel
(i) le lavage des premier et second acides nucléiques à code-barres à partir du support solide planaire a lieu avant la ligation et la ligation a lieu dans une solution lavée à partir du support solide planaire ; ou
(ii) la ligation a lieu dans une solution sur le support solide planaire et le lavage des premier et second acides nucléiques à code-barres à partir du support solide planaire a lieu après la ligation et avant le comblement de brèche(s).

14. Procédé selon la revendication 12 ou 13, dans lequel le procédé est répété pour une pluralité (par exemple, au moins 3, 5, 10, 20, 50, 100 ou plus) de billes liées à des extrémités 5' d'une pluralité d'oligonucléotides de codage à barres clonaux, les oligonucléotides de codage à barres comprenant une séquence de poignée PCR 5', une séquence de capture 3' et une séquence de code-barres unique à la bille à laquelle l'oligonucléotide à code-barres est lié, et dans lequel les oligonucléotides de pontage comprennent (i) une séquence terminale 3' complémentaire à la séquence de capture 3' des oligonucléotides de codage à barres clonaux et (ii) une séquence terminale 5' complémentaire à la portion 5' à un seul brin de l'oligonucléotide transposase, déterminant ainsi que des lectures de séquençage ayant des codes-barres à partir d'oligonucléotides de codage à barres amplifiés provenaient de billes adjacentes pour au moins une portion (par exemple, au moins 5 %, 10 %, 20 %, 40 %, 50 % ou plus) des billes sur la section tissulaire, générant ainsi une carte 2D de billes sur la section tissulaire.

15. Procédé selon l'une quelconque des revendications 12 à 14, dans lequel le marquage comprend en outre le marquage d'acides nucléiques dans la section tissulaire de sorte que deux types ou plus d'acides nucléiques soient étiquetés et ensuite séquencés, de préférence les deux types d'acides nucléiques sont l'ADN génomique ou l'ADNc.
